(19) Europäisches Patentamt
European Patent Office
Office européen des brevets

(11) **EP 3 463 268 B1**

(12) # EUROPEAN PATENT SPECIFICATION

(45) Date of publication and mention of the grant of the patent:
**03.11.2021 Bulletin 2021/44**

(21) Application number: **17736756.2**

(22) Date of filing: **23.05.2017**

(51) Int Cl.:
***A61K 8/35*** (2006.01) ***A61K 36/00*** (2006.01)
***A61K 8/9794*** (2017.01) ***A61K 8/9783*** (2017.01)
***A61K 41/00*** (2020.01) ***A61P 17/10*** (2006.01)
***A61K 8/22*** (2006.01) ***A61K 8/99*** (2017.01)
***A61K 8/97*** (2017.01) ***A61Q 19/08*** (2006.01)
***A61K 8/81*** (2006.01) ***A61K 8/49*** (2006.01)
***A61K 8/38*** (2006.01) ***A61K 8/36*** (2006.01)
***A61K 31/327*** (2006.01) ***A61K 33/40*** (2006.01)
***A61K 36/9066*** (2006.01) ***A61K 45/06*** (2006.01)
***C09B 61/00*** (2006.01) ***C09B 67/22*** (2006.01)

(86) International application number:
**PCT/IB2017/000726**

(87) International publication number:
**WO 2017/203359 (30.11.2017 Gazette 2017/48)**

(54) **BIOPHOTONIC COMPOSITIONS AND USES THEREOF**

BIOPHOTONISCHE ZUSAMMENSETZUNGEN UND VERWENDUNGEN DAVON

COMPOSITIONS BIOPHOTONIQUES ET UTILISATIONS DE CELLES-CI

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.05.2016 US 201662340371 P**

(43) Date of publication of application:
**10.04.2019 Bulletin 2019/15**

(73) Proprietor: **Klox Technologies Limited
Dublin 2 (IE)**

(72) Inventors:
- **LOUPIS, Nikolaos
14562 Athens (GR)**
- **PIERGALLINI, Remigio
63036 San Benedetto del Tronto (A.P.) (IT)**

(74) Representative: **BCF Global
Centre d'Entreprise et d'Innovation
56, Bd Niels Bohr
CS 52132
69603 Villeurbanne Cedex (FR)**

(56) References cited:
**WO-A1-2014/040177**
**WO-A1-2015/195458**
**WO-A2-03/017824**
**AU-A1- 2013 200 644**
**AU-A1- 2013 211 509**
**US-A1- 2004 126 442**
**US-A1- 2015 119 788**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 463 268 B1

## Description

## BACKGROUND OF THE DISCLOSURE

Phototherapy is recognized as having a wide range of applications in both the medical (pertaining to both humans and other animals) and cosmetic fields. For example, phototherapy has been used to disinfect target sites as an antimicrobial treatment, to promote wound healing, and for skin rejuvenation.

One type of phototherapy comprises the topical application to a target tissue of compositions comprising chromophores. When activated by an incident light, the chromophores absorb and emit light such as through fluorescence with a therapeutic effect on its own and/or in combination with the incident light also irradiating the target tissue. Furthermore, the light activated chromophore may react with an oxygen source to generate oxygen radicals such as singlet oxygen which at low levels may also have a therapeutic effect on the target tissue. Such compositions and used are disclosed in WO 2017/0410177, AU 2013 200 644 or US 2015/119788.

In another type of phototherapy, known as photodynamic therapy, a photosensitizer is applied to a target tissue and after a determined period of time during which the photosensitizer is absorbed by cells, the target tissue is exposed to a light source. The activated photosensitizer generates oxygen radicals from within the cells leading to cell destruction. Photodynamic therapy finds uses in cancer and antimicrobial treatments where cell destruction is a required mechanism of action.

It is the object of the present disclosure to provide improved compositions and methods useful in phototherapy.

## SUMMARY OF THE DISCLOSURE

The present disclosure provides a biophotonic composition comprising at least one photosynthetic organism-derived chromophore and at least one xanthene dye and a carrier medium wherein the at least one photosynthetic organism-derived chromophore is selected from: aloe-emodin, apigenin, berberine, caffeic acid, caffeine, curcumin, garcinia acid, gingerol, hyperforin, hypericin, ellagic acid, lycopene, oleuropein, piperine, resveratrol, sanguinarine, tannic acid, theobromine, zeaxanthin and combinations thereof, wherein the at least one photosynthetic organism-derived chromophore or the at least one xanthene dye is a fluorescent chromophore; and wherein the at least one photosynthetic organism-derived chromophore or the at least one xanthene dye has an emission spectrum that overlaps at least 20% with an absorption spectrum of the at least second chromophore.

In some embodiments of the foregoing or following, the composition further includes an oxidant or peroxide source. In certain such embodiments of the foregoing or following, the oxidant or peroxide source is selected from hydrogen peroxide, carbamide peroxide, benzoyl peroxide, peroxy acid, alkali metal peroxide, alkali metal percarbonate, peroxyacetic acid, alkali metal perborate, methyl ethyl ketone peroxide, or combinations thereof. In some embodiments, the peroxide is carbamide peroxide. The peroxide or peroxide precursor may be present in the biophotonic composition in an amount of about 0.01% to about 50% by weight of the final composition. In some embodiments, the composition does not include an oxidant or peroxide source.

In certain embodiments of the foregoing or following, the carrier medium comprises a hydrophilic polymer, a hygroscopic polymer, or a hydrated polymer, or combinations thereof. In some embodiments, the carrier medium is polyanionic in charge character. In some embodiments, the carrier medium comprises carboxylic functional groups. In some embodiments, the medium comprises a polymer having from 2 to 7 carbon atoms per functional group.

In certain embodiments of the foregoing or following, the carrier medium comprises a synthetic polymer selected from vinyl polymers, poly(ethylene oxide), acrylamide polymers, polyoxyethylene-polyoxypropylene copolymers, and derivatives or salts thereof and combinations thereof. In further embodiments, the carrier medium comprises one or more of a vinyl polymer selected from polyacrylic acid, polymethacrylic acid, polyvinyl pyrrolidone and polyvinyl alcohol. The carrier medium may comprise a carboxy vinyl polymer or a carbomer obtained by polymerization of acrylic acid. The carboxy vinyl polymer or carbomer may be crosslinked. In some embodiments, the carrier medium comprises Carbopol® 940, Carbopol® 980, ETD 2020 NF, Carbopol® 1382 Polymer, 71G NF, 971P NF, 974P NF, 980 NF, 981 NF, 5984 EP, ETF 2020 NF, ultrez 10 NF, ultrez 20, ultrez 21, 1342 NF, 934 NF, 934P NF, 940 NF, or 941 NF, or combinations thereof. In some embodiments, the carrier medium comprises 2-Hydroxyethyl methacrylate (HEMA) either alone or in addition to another carrier. In some embodiments, the 2-Hydroxyethyl methacrylate (HEMA) is added to the carrier medium in the form of microspheres or in a further physically reduced form such as in a finely ground particulate form or in a pulverized, powder form. In some embodiments the carrier medium comprises a polyacrylic acid polymer cross-linked with alkyl acrylate or allyl pentaerythritol. In some embodiments, the polymer is present in an amount of about 0.05% to about 5% by weight of the final composition, or about 0.1% to about 2.5%, or about 0.1% to about 2%, or about 0.5% to about 2.5%, or about 0.5% to about 2% by weight of the final composition. In some embodiments, the polymer is present in an amount of 0.05% to 5% by weight of the final composition, or 0.1% to 2.5%, or 0.1% to 2%, or 0.5% to 2.5%, or 0.5% to 2% by weight of the final composition.

In certain embodiments of the foregoing or following, the carrier medium comprises one or more protein-based polymers. In some embodiments, the protein-based polymer is gelatin, collagen, or both. In some embodiments the carrier medium comprises gelatin. In some embodiments, gelatin is present in an amount of equal to or more than about 4% by weight of the final composition, such as 4% by weight of the final composition. In other embodiments, the carrier medium comprises collagen. In some embodiments, collagen is present in an amount equal to or more than about 5% by weight of the final composition, such as 5% by weight of the final composition.

In certain embodiments of the foregoing or following, the carrier medium comprises sodium hyaluronate. In some embodiments, sodium hyaluronate is present in an amount of equal to or more than about 4% by weight of the final composition, such as 4% by weight of the final composition.

In certain embodiments of the foregoing or following, the carrier medium comprises one or more polysaccharides. In some embodiments, the polysaccharide is one or more of starch, chitosan, chitin, agar, alginates, xanthan, carrageenan, guar gum, gellan gum, pectin, or locust bean gum.

In some embodiments of the foregoing or following, the carrier medium comprises at least one glycol. In some embodiments, the glycol is one or more of ethylene glycol and propylene glycol.

In some embodiments of the foregoing or following, the carrier medium comprises a pharmaceutically acceptable medium.

In some implementations of the foregoing or following, the biophotonic compositions as defined herein further comprise a chromophore-protecting agent such as, but not limited to, a buffer, a salt, and a solvent that preserves the photochemical activity or property of the chromophore(s).

In some embodiments of the foregoing or following, the at least one photosynthetic organism-derived chromophore or the at least one xanthene dye absorbs and/or emits light within the range of about 400 nm to about 750 nm. The at least one photosynthetic organism-derived chromophore or the at least one xanthene dye may absorb and/or emit light within the green, orange and yellow portions of the electromagnetic spectrum. In some embodiments, the at least one photosynthetic organism- derived chromophore is Aloe-emodin. In some embodiments, the at least one photosynthetic organism-derived chromophore is Apigenin. In some embodiments, the at least one photosynthetic organism-derived chromophore is Berberine. In some embodiments, the at least one photosynthetic organism-derived chromophore is Caffeic acid. In some embodiments, the at least one photosynthetic organism-derived chromophore is Caffeine. In some embodiments, the at least one photosynthetic organism-derived chromophore is Curcumin. In some embodiments, the at least one photosynthetic organism-derived chromophore is Garcinia acid. In some embodiments, the at least one photosynthetic organism-derived chromophore is Gingerol. In some embodiments, the at least one photosynthetic organism-derived chromophore is Hyperforin. In some embodiments, the at least one photosynthetic organism-derived chromophore is Hypericin. In some embodiments, the at least one photosynthetic organism-derived chromophore is Ellagic Acid. In some embodiments, the at least one photosynthetic organism-derived chromophore is Lycopene. In some embodiments, the at least one photosynthetic organism-derived chromophore is Oleuropein. In some embodiments, the at least one photosynthetic organism-derived chromophore is Piperine. In some embodiments, the at least one photosynthetic organism- derived chromophore is Resveratrol. In some embodiments, the at least one photosynthetic organism-derived chromophore is Sanguinarine. In some embodiments, the at least one photosynthetic organism-derived chromophore is Tannic acid. In some embodiments, the at least one photosynthetic organism-derived chromophore is Theobromine. In some embodiments, the at least one photosynthetic organism-derived chromophore is Zeaxanthin. The structures of these chromophores are shown in Table 1:

Table 1

| NAME | STRUCTURE |
|---|---|
| Aloe-emodin | 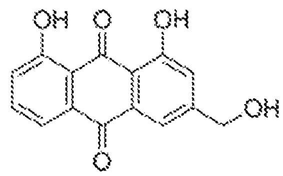 |
| Apigenin | 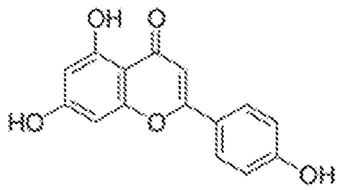 |

(continued)

| NAME | STRUCTURE |
|---|---|
| Berberine chloride hydrate | 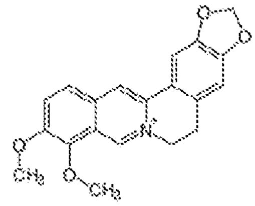 |
| Caffeic acid | 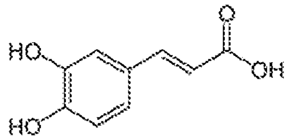 |
| Caffeic acid phenethyl ester (CAPE) | 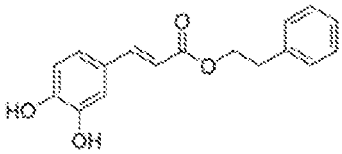 |
| Caffeine | 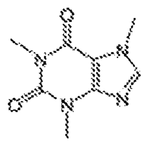 |
| Curcumin | 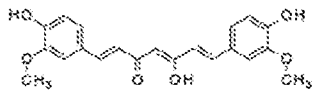 |
| Ellagic acid | 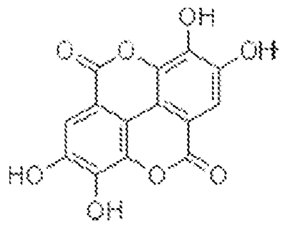 |
| Garcinia acid | 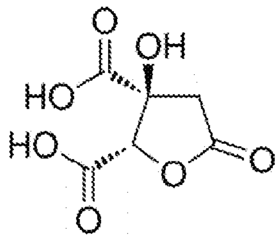 |
| 10-Gingerol | 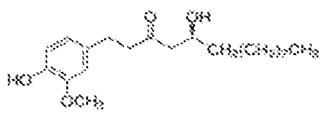 |
| Hyperforin | 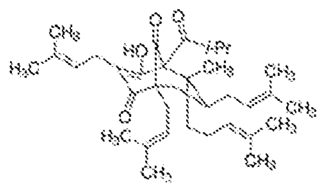 |

(continued)

| NAME | STRUCTURE |
|---|---|
| Hypericin | 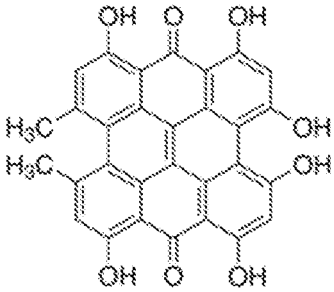 |
| Lycopene | 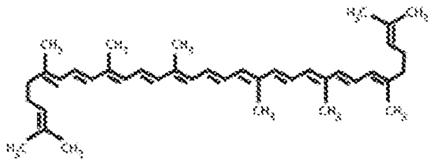 |
| Oleuropein | 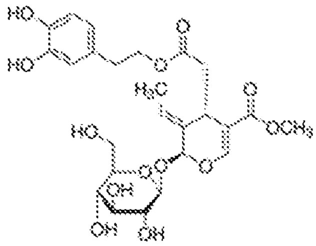 |
| Piperine | 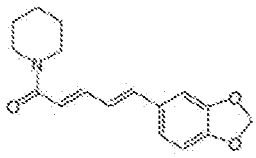 |
| Resveratrol | 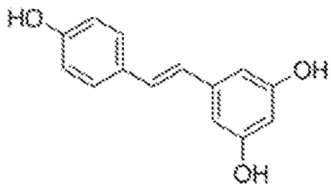 |
| Sanguinarine chloride hydrate | 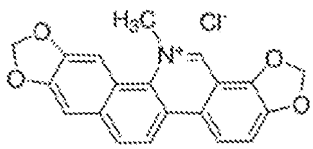 |
| Tannic acid | 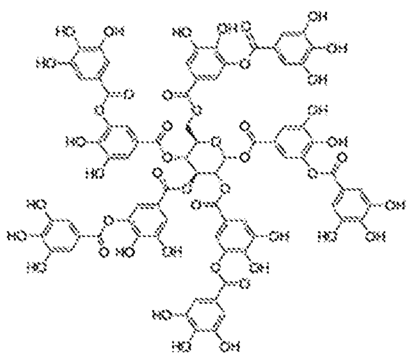 |

(continued)

| NAME | STRUCTURE |
|---|---|
| Theobromine | 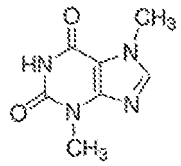 |
| Zeaxanthin | 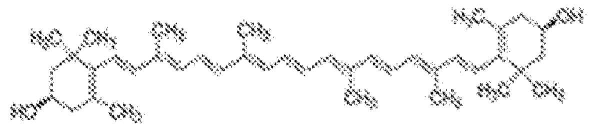 |

In certain embodiments, the xanthene dye is Eosin Y, Eosin B, Erythrosin B, Fluorescein, Rose Bengal, Phloxin B, or combinations thereof. In some embodiments, the xanthene dye is a combination of Eosin Y and Rose Bengal. In some embodiments, the xanthene dye is Eosin Y. In some embodiments, the xanthene dye is Rose Bengal. In further implementations, the at least one photosynthetic organism-derived chromophore or the at least one xanthene dye has an emission spectrum that overlaps with an absorption spectrum of the at least second chromophore. In further embodiments, the at least one photosynthetic organism-derived chromophore or molecular complex or the at least one xanthene dye or molecular complex has an emission spectrum that overlaps at least 20% with an absorption spectrum of the at least second chromophore. The at least one photosynthetic organism-derived chromophore or molecular complex or the at least one xanthene dye or molecular complex may transfer energy to the at least second chromophore upon illumination with a light.

In certain embodiments of the foregoing or following, the biophotonic composition has a translucency of at least about 40%, about 45%, about 50%, about 55%, about 60%, about 65%, about 70%, about 75%, about 80%, about 85%, about 90%, about 91%, about 92%, about 93%, about 94%, about 95%, about 96%, about 97%, about 98%, about 99%, or about 100% in a visible range when measured without the chromophore(s) present. In some embodiments of the foregoing or following, the biophotonic composition has a translucency of at least 40%, 45%, 50%, 55%, 60%, 65%, 70%, 75%, 80%, 85%, 90%, 91%, 92%, 93%, 94%, 95%, 96%, 97%, 98%, 99%, or 100% in a visible range when measured without the chromophore(s) present.

In certain embodiments of the foregoing or following, the biophotonic composition is used in cosmetic or for use for the medical treatment of a tissue or bone. In some embodiments, the cosmetic treatment includes skin rejuvenation and conditioning. In some embodiments, the medical treatment includes wound healing, periodontitis treatment, and treatment of a skin condition. The skin condition may be acne, eczema, psoriasis or dermatitis. In some embodiments, the biophotonic composition is for use for modulating inflammation. In some embodiments, the biophotonic composition is for use for modulating collagen production. In other embodiments, the biophotonic composition is for use for promoting angiogenesis. In some embodiments, the biophotonic composition is used for loosing or removing dry or dead skin. In some embodiments, the biophotonic composition is for use for treating bacterial, viral or fungal infections. In some embodiments, the biophotonic composition is for use for debridement of wounds or skin. In some embodiments, the medical treatment includes tissue repair, bone injury or disease repair, wound healing, oral disease treatment, periodontitis treatment, treatment of bacterial, viral or fungal infections, treatment of a fistula, or treatment of a skin condition.

In some embodiments, upon exposure to light, the biophotonic composition emits at least 25% to at least 99% more red, yellow and/or orange light than a composition lacking the at least one photosynthetic organism-derived chromophore or the at least one non- photosynthetic prokaryote-derived chromophore. In some embodiments, upon exposure to light, the biophotonic composition emits at least 1.25x, 1.5x, 1.75x or more red, yellow and/or orange light than a composition lacking the at least one photosynthetic organism- derived chromophore or the at least one xanthene dye. In other embodiments, upon exposure to light, the composition emits at least 5x, 10x or 20x more red, yellow and/or orange light than a composition lacking the at least one photosynthetic organism-derived chromophore or the at least one xanthene dye.

The light that may be useful for illumination of the biophotonic composition as defined herein is a continuous light. In some other implementations, the light that may be useful for illumination of the biophotonic composition as defined herein is a modulated light such as a pulsed light. In some implementations of this aspect, the light source that may be useful for illumination of the biophotonic composition as defined herein is a light-emitting diode (LED).

## DETAILED DESCRIPTION

*(1) Definitions*

Before continuing to describe the present disclosure in further detail, it is to be understood that this disclosure is not limited to specific compositions or process steps, as such may vary. It must be noted that, as used in this specification and the appended claims, the singular form "a", "an" and "the" include plural referents unless the context clearly dictates otherwise.

As used herein, the term "about" in the context of a given value or range refers to a value or range that is within 20%, preferably within 10%, and more preferably within 5% of the given value or range.

It is convenient to point out here that "and/or" where used herein is to be taken as specific disclosure of each of the two specified features or components with or without the other. For example "A and/or B" is to be taken as specific disclosure of each of (i) A, (ii) B and (iii) A and B, just as if each is set out individually herein.

"Biophotonic" means the generation, manipulation, detection and application of photons in a biologically relevant context. In other words, biophotonic compositions exert their physiological effects primarily due to the generation and manipulation of photons, for example, by absorbing photon to emit photons or to transfer energy, for example, by absorbing photons to emit photons or to transfer energy.

Terms "chromophore", "photoactivating agent", and "photoactivator" are used herein interchangeably. A chromophore means a chemical compound, when contacted by light irradiation, is capable of absorbing the light. The chromophore(s) readily undergoes photoexcitation and can transfer its energy to other molecules or emit it as light (e.g. fluorescence).

The term "actinic light" is intended to mean light energy emitted from a specific light source (e.g. lamp, LED, laser or sunlight) and capable of being absorbed by matter (e.g. the chromophore(s) or photoactivator(s)). The expression "actinic light" and the term "light" are used herein interchangeably. In some embodiments, the actinic light is visible light.

The term "oxidant" is intended to mean either a compound that readily transfers oxygen atoms and oxidizes other compounds, or a substance that gains electrons in a redox chemical reaction.

The term "photosynthetic organism-derived" is intended to mean a compound derived from an organism capable of photosynthesis.

The term "reactive oxygen species" is intended to mean chemically-reactive molecules containing oxygen. Examples include oxygen ions and peroxides. They can be either inorganic or organic. Active oxygen species are highly reactive due to the presence of unpaired valence shell electrons. They are also referred to as "reactive oxygen", "active oxygen", or "active oxygen species".

"Topical application", "topical", or "topical uses" means application to body surfaces, such as the skin, mucous membranes, vagina, oral cavity, internal surgical wound sites, and the like.

"Skin rejuvenation" means a process of reducing, diminishing, retarding or reversing one or more signs of skin aging or generally improving the condition of skin. For instance, skin rejuvenation may include increasing luminosity of the skin, reducing pore size, reducing fine lines or wrinkles, improving thin and transparent skin, improving firmness, improving sagging skin (such as that produced by bone loss), improving dry skin (which might itch), reducing or reversing freckles, reducing or preventing the appearance of age spots, spider veins, rough and leathery skin, fine wrinkles that disappear when stretched, reducing loose skin, or improving a blotchy complexion. According to the present disclosure, one or more of the above conditions may be improved or one or more signs of aging may be reduced, diminished, retarded or even reversed by certain embodiments of the compositions, methods and uses of the present disclosure. "Wound" means an injury to any tissue including, for example, acute, subacute, delayed or difficult to heal wounds, and chronic wounds. Examples of wounds may include both open and closed wounds. Wounds include, for example, amputations, burns, incisions, excisions, lesions, lacerations, abrasions, puncture or penetrating wounds, surgical wounds, amputations, contusions, hematomas, crushing injuries, ulcers (such as for example pressure, diabetic, venous or arterial), scarring (cosmesis), wounds caused by periodontitis (inflammation of the periodontium).

(2) Biophotonic Compositions Comprising Photosynthetic Organism-Derived Chromophores andXanthene dyes

The present disclosure provides, in a broad sense, biophotonic compositions which can be activated by light (e.g., photons) of specific wavelengths. A biophotonic composition according to various embodiments of the present disclosure contains at least one photosynthetic organism-derived chromophore and at least one xanthene dye, or a molecular complex comprising the at least one photosynthetic organism-derived chromophore and the at least one xanthene dye, within a carrier medium. Activation of the chromophore(s) in the biophotonic composition may lead to the generation of oxygen radicals such as singlet oxygen, and as at least one of the chromophore(s) is a fluorophore, it also leads to the generation of light of a different wavelength, each one of which individually or together may have a therapeutic effect.

When a chromophore absorbs a photon of a certain wavelength, it becomes excited. This is an unstable condition and the molecule tries to return to the ground state, giving away the excess energy. For some chromophores, it is favorable to emit the excess energy as light when returning to the ground state. This process is called fluorescence. The peak wavelength of the emitted fluorescence is shifted towards longer wavelengths compared to the absorption wavelengths due to loss of energy in the conversion process. This is called the Stokes' shift. In the proper environment (e.g., in a biophotonic composition) much of this energy is transferred to the other components of the biophotonic composition or to the treatment site directly. Without being bound to theory, it is thought that fluorescent light emitted by photoactivated chromophores may have therapeutic properties due to its femto-, pico-, or nano-second emission properties which may be recognized by biological cells and tissues, leading to favorable biomodulation. Furthermore, the emitted fluorescent light has a longer wavelength and hence a deeper penetration into the tissue than the activating light. Irradiating tissue with such a broad range of wavelength, including in some embodiments the activating light which passes through the composition, may have different and complementary effects on the cells and tissues. In other words, chromophores are used in the biophotonic compositions of the present disclosure for therapeutic effect on tissues. This is a distinct application of these photoactive agents and differs from the use of chromophores as simple stains or as catalysts for photo-polymerization. The biophotonic compositions of the present disclosure may be described based on the components making up the composition. Additionally or alternatively, the compositions of the present disclosure have functional and structural properties and these properties may also be used to define and describe the compositions. Individual components of the biophotonic compositions of the present disclosure, including chromophores, oxidants (peroxides and peroxide precursors), carrier mediums and other optional ingredients, are detailed below.

(a) Chromophores

The biophotonic compositions and compositions for uses of the present disclosure comprise at least one photosynthetic organism-derived chromophore and at least one xanthene dye. In some embodiments, the at least one photosynthetic organism-derived chromophore or the at least xanthene dye
absorbs at a wavelength in the range of the visible spectrum, such as at a wavelength of about 380 nm-800 nm, about 380 nm-700 nm, about 400 nm-800 nm, or about 380 nm-600 nm. In other embodiments, the at least one photosynthetic organism-derived chromophore or the at least one xanthene dye absorbs at a wavelength of about 200 nm-800 nm, about 200 nm-700 nm, about 200 nm-600 nm or about 200 nm-500 nm. In some embodiments, the at least one photosynthetic organism- derived chromophore or the at least one xanthene dye absorbs at a wavelength of about 200 nm-600 nm. In some embodiments, the at least one photosynthetic organism-derived chromophore or the at least one xanthene dye absorbs light at a wavelength of about 200 nm-300 nm, about 250 nm-350 nm, about 300 nm-400 nm, about 350 nm-450 nm, about 400 nm-500 nm, about 450 nm-650 nm, about 600 nm-700 nm, about 650 nm-750 nm or about 700 nm-800 nm. In some embodiments, the at least one photosynthetic organism-derived chromophore or the at least one xanthene dye absorbs at a wavelength of 380 nm-800 nm, 380 nm-700 nm, 400 nm-800 nm, or 380 nm-600 nm. In other embodiments, the at least one photosynthetic organism-derived chromophore or the at least one xanthene dye absorbs at a wavelength of 200 nm- 800 nm, 200 nm-700 nm, 200 nm-600 nm or 200 nm-500 nm. In some embodiments, the at least one photosynthetic organism-derived chromophore or the at least one non- photosynthetic prokaryote-derived chromophore absorbs at a wavelength of 200 nm-600 nm. In some embodiments, the at least one photosynthetic organism-derived chromophore or the at least one xanthene dye absorbs light at a wavelength of 200 nm-300 nm, 250 nm-350 nm, 300 nm-400 nm, 350 nm-450 nm, 400 nm- 500 nm, 450 nm-650 nm, 600 nm-700 nm, 650 nm-750 nm or 700 nm-800 nm.

It will be appreciated to those skilled in the art that optical properties of a particular chromophore may vary depending on the chromophore's surrounding medium. Therefore, as used herein, a particular chromophore's absorption and/or emission wavelength (or spectrum) corresponds to the wavelengths (or spectrum) measured in a biophotonic composition of the present disclosure.

In some embodiments, the at least one photosynthetic organism-derived chromophore is obtained from a plant extract, for example, but not limited to, extracts of coffee beans, green tea leaves, blueberries, cranberries, huckleberries, acai berries, goji berries, blackberries, raspberries, grapes, strawberries, persimmon, pomegranate, lingonberry, bearberry, mulberry, bilberry, choke cherry, sea buckthorn berries, goji berry, tart cherry, kiwi, plum, apricot, apple, banana, berry, blackberry, blueberry, cherry, cranberry, currant, greengage, grape, grapefruit, gooseberry, lemon, mandarin, melon, orange, pear, peach, pineapple, plum, raspberry, strawberry, sweet cherry, watermelon, wild strawberry, and pips (seeds) of fig of barbarism (fruit of a cactus found in the Mediterranean and North Africa, recognizable by its thorns). In some embodiments, the at least one photosynthetic organism-derived chromophore is obtained from trees, including for instance sequoia, coastal redwood, bristlecone pine, birch, and cedar.

In some embodiments, the at least one photosynthetic organism-derived chromophore is obtained from leafy or salad vegetables (e.g., Amaranth *(Amaranthus cruentus),* Arugula (*Eruca sativa*), Beet greens *(Beta vulgaris* subsp. *vulgaris),* Bitterleaf *(Vernonia calvoana*), Bok choy (*Brassica rapa Chinensis* group), Broccoli Rabe *(Brassica rapa* subsp.

*rapa*), Brussels sprout *(Brassica oleracea Gemmifera* group), Cabbage (*Brassica oleracea Capitata* group), Catsear (*Hypochaeris radicata*), Celery (*Apium graveolens),* Celtuce (*Lactuca sativa* var. *asparagina*), Ceylon spinach (*Basella alba*), Chard *(Beta vulgaris* var. *cicla*), Chaya (*Cnidoscolus aconitifolius* subsp. *aconitifolius*), Chickweed (*Stellaria*), Chicory *(Cichorium intybus),* Chinese cabbage *(Brassica rapa Pekinensis* group), Chinese Mallow (*Malva verticillata*), Chrysanthemum leaves *(Chrysanthemum coronarium),* Collard greens *(Brassica oleracea),* Corn salad (*Valerianella locusta*), Cress (*Lepidium sativum*), Dandelion (*Taraxacum officinale),* Endive *(Cichorium endivia*), Epazote *(Chenopodium ambrosioides),* Fat hen *(Chenopodium album),* Fiddlehead (*Pteridium aquilinum, Athyrium esculentum),* Fluted pumpkin (*Telfairia occidentalis),* Garden Rocket (*Eruca sativa*), Golden samphire (*Inula crithmoides*), Good King Henry *(Chenopodium bonus-henricus*), Greater Plantain *(Plantago major),* Kai-lan (*Brassica rapa Alboglabra* group), Kale *(Brassica oleracea Acephala* group), Komatsuna *(Brassica rapa Pervidis* or *Komatsuna* group), Kuka (*Adansonia* spp.), Lagos bologi (*Talinum fruticosum*), Land cress (*Barbarea verna*), Lettuce (*Lactuca sativa*), Lizard's tail (*Houttuynia cordata),* Melokhia *(Corchorus olitorius, Corchorus capsularis),* Mizuna greens *(Brassica rapa Nipposinica* group), Mustard *(Sinapis alba*), New Zealand Spinach (*Tetragonia tetragonioides),* Orache (*Atriplex hortensis),* Paracress (*Acmella oleracea),* Pea sprouts/leaves *(Pisum sativum),* Polk (*Phytolacca americana*), Radicchio *(Cichorium intybus),* Samphire (*Crithmum maritimum*), Sea beet *(Beta vulgaris* subsp. *maritima),* Seakale *(Crambe maritima*), Sierra Leone bologi (*Crassocephalum* spp.), Soko *(Celosia argentea*), Sorrel (*Rumex acetosa*), Spinach (*Spinacia oleracea*), Summer purslane (*Portulaca oleracea),* Swiss chard (*Beta vulgaris* subsp. *cicla var. flavescens*), Tatsoi *(Brassica rapa Rosularis* group), Turnip greens *(Brassica rapa Rapifera* group), Watercress *(Nasturtium officinale*), Water spinach *(Ipomoea aquatica*), Winter purslane (*Claytonia perfoliata*), Yarrow (*Achillea millefolium*)); fruiting and flowering vegetables, such as those from trees (e.g., Avocado *(Persea americana*), Breadfruit *(Artocarpus altilis*)); or from annual or perennial plants (e.g., Acorn squash *(Cucurbita pepo*), Armenian cucumber *(Cucumis melo Flexuosus* group), Aubergine *(Solanum melongena*), Bell pepper *(Capsicum annuum),* Bitter melon (*Momordica charantia*), Caigua *(Cyclanthera pedata),* Cape Gooseberry (*Physalis peruviana*), Capsicum *(Capsicum annuum),* Cayenne pepper (*Capsicum frutescens),* Chayote *(Sechium edule),* Chili pepper (*Capsicum annuum Longum* group), Courgette (*Cucurbita pepo),* Cucumber *(Cucumis sativus),* Eggplant *(Solanum melongena),* Luffa (*Luffa acutangula, Luffa aegyptiaca),* Malabar gourd *(Cucurbita ficifolia*), Parwal (*Trichosanthes dioica*), Pattypan squash *(Cucurbita pepo),* Perennial cucumber (*Coccinia grandis*)*,* Pumpkin (*Cucurbita maxima, Cucurbita pepo),* Snake gourd (*Trichosanthes cucumerina),* Squash aka marrow *(Cucurbita pepo*); Sweet corn aka corn, aka maize *(Zea mays),* Sweet pepper (*Capsicum annuum Grossum* group), Tinda *(Praecitrullus fistulosus*), Tomatillo (*Physalis philadelphica*), Tomato *(Lycopersicon esculentum* var), uva ursi (*Arctostaphylos uva-ursi),* Winter melon (*Benincasa hispida*), West Indian gherkin *(Cucumis anguria*), Zucchini *(Cucurbita pepo*)); the flower buds of perennial or annual plants (e.g., Artichoke (*Cynara cardunculus, C. scolymus),* Broccoli (*Brassica oleracea*), Cauliflower *(Brassica oleracea), Calendula,* henna, Squash blossoms (*Cucurbita* spp.); podded vegetables (e.g., American groundnut (*Apios americana),* Azuki bean (*Vigna angularis*), Black-eyed pea *(Vigna unguiculata* subsp. *unguiculata*), Chickpea *(Cicer arietinum*), Common bean *(Phaseolus vulgaris),* Drumstick *(Moringa oleifera*), Dolichos bean (*Lablab purpureus*), Fava bean (*Viciafaba*), Green bean *(Phaseolus vulgaris),* Guar *(Cyamopsis tetragonoloba*), Horse gram (*Macrotyloma uniflorum),* Indian pea *(Lathyrus sativus*), Lentil (*Lens culinaris*), Lima Bean *(Phaseolus lunatus*), Moth bean (*Vigna acontifolia*), Mung bean *(Vigna radiata),* Okra *(Abelmoschus esculentus),* Pea *(Pisum sativum),* Peanut *(Arachis hypogaea*), Pigeon pea *(Cajanus cajan*), Ricebean (*Vigna umbellata*), Runner bean *(Phaseolus coccineus*), Soybean *(Glycine max*), Tarwi (*tarhui, chocho; Lupinus mutabilis),* Tepary bean *(Phaseolus acutifolius*), Urad bean *(Vigna mungo*), Velvet bean *(Mucuna pruriens),* Winged bean *(Psophocarpus tetragonolobus),* Yardlong bean (*Vigna unguiculata* subsp. *sesquipedalis));* bulb and stem vegetables (e.g., Asparagus *(Asparagus officinalis),* Cardoon (*Cynara cardunculus),* Celeriac *(Apium graveolens* var. *rapaceum),* Celery *(Apium graveolens),* Elephant Garlic *(Allium ampeloprasum* var. *ampeloprasum*), Florence fennel (*Foeniculum vulgare* var. *dulce),* Garlic *(Allium sativum),* Kohlrabi (Brassica oleracea Gongylodes group), Kurrat *(Allium ampeloprasum* var. *kurrat*), Leek *(Allium porrum*), Lotus root *(Nelumbo nucifera*), Nopal (*Opuntiaficus-indica*), Onion *(Allium cepa*), Prussian asparagus (*Ornithogalum pyrenaicum*), Shallot *(Allium cepa Aggregatum* group), Welsh onion (*Allium fistulosum*), Wild leek *(Allium tricoccum*)); root and tuberous vegetables (e.g., Ahipa (*Pachyrhizus ahipa*), Arracacha *(Arracacia xanthorrhiza*), Bamboo shoot (*Bambusa vulgaris* and *Phyllostachys edulis*), Beetroot (*Beta vulgaris* subsp. *vulgaris),* Black cumin *(Bunium persicum),* Burdock (*Arctium lappa*), Broadleaf arrowhead (*Sagittaria latifolia*), Camas (*Camassia*), Canna (*Canna* spp.), Carrot *(Daucus carota*), Cassava (*Manihot esculenta*), Chinese artichoke *(Stachys affinis),* Daikon *(Raphanus sativus Longipinnatus* group), Earthnut pea *(Lathyrus tuberosus),* Elephant Foot yam (*Amorphophallus paeoniifolius*), Ensete *(Ensete ventricosum*), Ginger *(Zingiber officinale),* Gobo (*Arctium lappa*), Hamburg parsley (*Petroselinum crispum* var. *tuberosum*), Jerusalem artichoke *(Helianthus tuberosus*), Emma (*Pachyrhizus erosus),* Parsnip (*Pastinaca sativa),* Pignut (*Conopodium majus),* Plectranthus *(Plectranthus* spp.), Potato *(Solanum tuberosum),* Prairie turnip (*Psoralea esculenta*), Radish *(Raphanus sativus),* Rutabaga (*Brassica napus Napobrassica* group), Salsify *(porrifolius*), Scorzonera (*Scorzonera hispanica),* Skirret (*Sium sisarum),* Sweet Potato or Kumara *(Ipomoea batatas),* Taro (*Colocasia esculenta*), Ti *(Cordyline fruticosa*), Tigernut *(Cyperus esculentus),* Turnip *(Brassica rapa Rapifera* group), Ulluco *(Ullucus tuberosus),* Wasabi (*Wasabia japonica*), Water chestnut (*Eleocharis dulcis),* Yacon (*Smallanthus sonchifolius*), Yam *(Dioscorea* spp.)); spices and other flavorings

(e.g., ajowan *(Trachyspermum ammi*) allspice *(Pimenta dioica*), amchur *(Mangifera indica),* angelica *(Angelica* spp.), anise (*Pimpinella anisum*), annatto (*Bixa orellana*), asafoetida (*Ferula asafoetida), Astragalus,* barberry *(Berberis* spp (many) and *Mahonia* spp (many)), basil *(Ocimum* spp)., bay leaf *(Laurus nobilis),* bee balm (bergamot, monarda; *Monarda* spp.), black cumin *(Bunium persicum),* black lime (loomi; *Citrus aurantifolia*), boldo (boldina; *Peumus boldus*), bush tomato (akudjura; *Solanum central),* borage *(Borago officinalis*), calamus (sweet flag; *Acorus calamus),* candlenut *(Aleurites moluccana),* caraway *(Carum carvi),* cardamom *(Amomum compactum*), capers *(Capparis spinosa*), cassia (*Cimmanmomum cassia),* cayenne pepper *(Capsicum annum*), celery *(Apium graveolens),* chervil *(Anthriscus cerefolium),* chicory *(Cicorium intybus),* chile/chili/chilli (e.g., *Capsicum frutescens*), chile varieties (*Capsicum frutescens*), chives *(Allium odorum, Allium shoenoprasum*), cilantro *(Coriandrum sativum),* cinnamon (*Cinnamomum zeylanicum; Cinnamomum cassia),* clove *(Syzygium aromaticum*), coriander (Coriandrum sativum), cubeb *(Piper cubeba*), cumin *(Cuminum cyminum*), curry leaf (kari; *Murraya koenigii*), dill (*Anethum graveolens),* elder (elder flower, & elderberry; *Sambucus nigra*), epazote *(Chenopodium ambrosioides*), fennel (*Foeniculum vulgare),* fenugreek (*Trigonella foenum-graecum*), galangal (*Alpinia galangal*), garlic *(Allium sativum),* ginger (*Zingiber officinale*), (*Lawsonia inermis*), ginseng, hoja santa *(Piper auritum*), horseradish (*Armoracia rusticana*), hyssop (*Hyssopus officinalis),* jamaican sorrel *(Hibiscus sabdariffa*), juniper *(Juniperus communis),* kaffir lime (*Citrus hystrix),* mustard (*Brassica nigra),* kokum (*Garcinia indica*), lavender *(Lavandula angustifolia*), lemon balm (*Melissa officinalis),* lemon grass (*Cymbopogon citrates*), lemon myrtle *(Backhousia citriodora*), lemon verbena (*Lippia citriodora*), licorice *(Glycyrrhiza glabra*), lovage (*Levisticum officinale),* mace (*Myristica fragrans*), mahlab (*Prunus mahaleb),* marjoram *(Majorana hortensis),* mastic (*Pistacia lenticus),* melegueta pepper (*Aframomum melegueta),* grains of paradise *(Aframomum granum paradise*), mint (*Mentha* spp.), mountain pepper (*Tasmannia lanceolata),* Tasmanian pepper (*Tasmannia lanceolata*), myrtle (*Myrtus communis),* nigella *(Nigella sativa*), nutmeg (*Myristica fragrans*), onion *(Allium cepa*), orris root *(Germanica florentina),* paprika (*Capsicum annuum*), parsley *(Petroselinum crispum),* pepper *(Piper nigrum),* poppy seed (*Papaver somniferum), Reseda,* rosemary (*Rosmarinus officinalis),* saffron *(Crocus sativus*), sage (*Salvia officinalis*), sassafras *(Sassafras albidum*), savory (*Satureja hortensis*), scented geranium *(Pelargonium* spp), screw-pine (pandan; *Pandanus tectorius*), sesame *(Sesamum indicum),* soapwort (*Saponaria officinalis*), sorrel (*Rumex acetosa*), star anise (*Illicium verum*), sumac (*Rhus coriaria*), szechwan pepper (*Zanthoxylum* spp. *(piperitum, simulans, bungeanum, rhetsa acanthopodium*)), tamarind *(Tamarindus indica*), tarragon (*Artemisia dracunculus),* thyme (*Thymus vulgaris),* turmeric *(Curcuma longa*), vanilla (*Vanilla planifolia*), wasabi (*Wasabia japonica),* watercress (*Nasturtium officinale),* wattleseed (*Acacia aneuro*), zedoary (*Curcuma zedoaria*), and sea vegetables and algae (e.g., Aonori (*Monostroma* spp., *Enteromorpha* spp.), Brown algae (*Phaeophyceae*), Carola (*Callophyllis variegata),* Dabberlocks aka badderlocks (*Alaria esculenta*), Dulse (*Palmaria palmata*), Gim (*Porphyra* spp.), Hijiki (*Hizikia fusifivrmis*), Kombu (*Laminaria japonica),* Laver *(Porphyra* spp.), Mozuku (*Cladosiphon okamuranus),* Nori (*Porphyra* spp.), Ogonori (*Gracilaria* spp.), Sea grape (*Caulerpa* spp.), Seakale *(Crambe maritima*), Sea lettuce (*Ulva lactuca),* Wakame (*Undaria pinnatifida*)), some of which are not plants in the taxonomic sense.

In some embodiments, the at least one photosynthetic organism-derived chromophore is obtained from the genus *Curcuma.* In some embodiments, the at least one photosynthetic organism-derived chromophore is obtained from the species *Curcuma longa* or *Curcuma zedoria.*

In some embodiments, the photosynthetic oiganism-derived chromophore is selected from, but is not limited to, Aloe-emodin, Apigenin, Berberine, Caffeic acid, Caffeine, Curcumin, Gingerol, Hyperforin, Hypericin, Ellagic Acid, Lycopene, Oleuropein, Piperine, Resveratrol, Sanguinarine, Tannic acid, Theobromine, and Zeaxanthin.

Aloe emodin can be obtained from the gel, sap or leaves of aloe vera, the bark of Frangula (*Rhamnus frangula*) and Cascara Sagrada *(Rhamnus purshiana*), the leaves of Senna (*Cassia angustifolia*), and the rhizome of Rhubarb *(Rheum rhaponticum).* Emodin can also be found in plant species including, but not limited to, the following: *Acalypha australis, Cassia occidentalis, Cassia siamea, Fallopia japonica, Flossostemon bruguieri, Kalimeris indica, Polygonum hypoleucum, Rhamnus alnifiolia, Rhamnus cathartica, Rheum palmatum, Rumex nepalensis, Senna obtusifolia, Thielavia subthermophila, and Ventilago madraspatana.*

Apigenin (4',5,7-trihydroxyflavone), found in many plants, is a natural product belonging to the flavone class that is the aglycone of several naturally occurring glycosides. Apigenin is found in many fruits and vegetables, however parsley, celery and chamomile tea are the most common sources.

Berberine is a quaternary ammonium salt from the protoberberine group of isoquinoline alkaloids. It is found in such plants as Berberis (e.g., *Berberis aquifolium* (Oregon grape), *Berberis vulgaris* (barberry), *Berberis aristata* (tree turmeric)), *Hydrastis canadensis* (goldenseal), *Xanthorhiza simplicissima* (yellowroot), *Phellodendron amurense* (Amur cork tree), *Coptis chinensis* (Chinese goldthread or Huang Lian Su), *Tinospora cordifolia, Argemone mexicana* (prickly poppy), and *Eschscholzia californica* (Californian poppy). Berberine is usually found in the roots, rhizomes, stems, and bark.

Caffeic acid can be found in the bark of *Eucalyptus globulus.* It can also be found in the freshwater fern *Salvinia molesta* or in the mushroom *Phellinus linteus.*

Caffeine is a xanthine alkaloid. Caffeine is found in varying quantities in the seeds, leaves, and fruit of some plants, such as Guarana, Yerba Mate, Cola Nut, and Cacao.

Curcumin is a diarylheptanoid. It is the principal curcuminoid of the spice turmeric, which is a member of the ginger family (*Zingiberaceae*).

Gingerol can be isolated from the rhizomes or roots of the plant *Zingiber officinale* (ginger).

Hyperforin is a prenylated phloroglucinol derivative. It can be isolated from members of the plant genus *Hypericum,* notably *Hypericum perforatum* (St John's wort).

Hypericin is a naphthodianthrone, a red-colored anthraquinone-derivative, which, together with hyperforin, is one of the principal active constituents of *Hypericum* (Saint John's wort).

Ellagin acid is a polyphenol compound found in blackberries, cranberries, pecans, pomegranates, raspberries, strawberries, walnuts, wolfberries, and grapes.

Lycopene is a bright red carotene and carotenoid pigment found in tomatoes and other red fruits and vegetables, such as red carrots, watermelons, gac, and papayas (but not strawberries, red bell peppers, or cherries).

Oleuropein is a phenylethanoid, a type of phenolic compound found in olive leaf from the olive tree.

Piperine, along with its isomer chavicine, is an alkaloid and is present in black pepper and long pepper.

Theobromine is an alkaloid of the cacao plant. It is classified as a xanthine alkaloid, which also includes the similar compounds theophylline and caffeine. Plants from which Theobromine can be obtained include *Theobroma cacao, Theobroma bicolor, Ilex paraguariensis, Camellia sinensis, Cola acuminate, Theobroma angustifolium, Guarana,* and *Coffea Arabica.*

Resveratrol is found in the skin of red grapes and in other fruits as well as in the roots of Japanese knotweed (Polygonum cuspidatum).

Sanguinarine is a quaternary ammonium salt from the group of benzylisoquinoline alkaloids. It can be extracted from plants, including bloodroot *(Sanguinaria canadensis),* Mexican prickly poppy *Argemone mexicana, Chelidonium majus* and *Macleaya cordata.* It is also found in the root, stem and leaves of the opium poppy. Tannic acid is a type of polyphenol and can be extracted from plants, for example, *Caesalpinia spinosa, Rhus semialata, Quercus infectoria* and *Rhus coriaria*. Zeaxanthin is a carotenoid and can be extracted from paprika, corn, saffron, wolfberries and many other plants. Additional examples of photosynthetic organism-derived chromophores include, but are not limited to, phloroglucinols, adhyperforin, terpenoids, polyphenols, capsaicin, stilbenoids, flavonoids, catechins, capsaicinoids, alkaloids, quinones, ketides, tannins, antraquinones, iridoids, curcuminoids, furocoumarins, phytosterols, carotenoids, isothiocyanates, ginsenosides, withanolides, and derivatives thereof.

In some embodiments, at least one xanthene dye is derived from bacteria including, but not limited to, *Agrobacterium aurantiacum; Paracoccus carotinifaciens; Bradyrhizobium sp.; Flavobacterium sp., Paracoccus zeaxanthinifaciens; Achromobacter; Bacillus; Brevibacterium sp.; Corynebacterium michigannise; Corynebacterium insidiosum; Rugamonas rubra; Streptoverticillium rubrireticuli; Vibrio gaogenes; Alteromonas rubra; Rhodococcus maris; Xanthophyllomyces dendrorhous; Haloferax alexandrines; Staphylococcus aureus; Chromobacterium violaceum; Serratia marcescens; Serratia rubidaea; Pseudomonas aeruginosa; Xanthomonas oryzae; Janthinobacterium lividum; Streptoverticillium rubrireticuli; Streptomyces echinoruber; Arthrobacter; Chromobacterium sp.; Micrococcus sp.; Rheinheimera sp.; Sphingobacterium; Actinobacteria; Flavobacterium; Chryseobacterium; Pseudoalteromonas; Altermonas denitrificans; Hahella; Vibrio; Alteromonas luteoviolacea;* and *Janthinobacterium.*

In some embodiments, at least one xanthene dye includes, but is not limited to, Astaxanthin, Canthaxanthin, Zeaxanthin, Indigoidine, Prodigiosin, Staphyloxanthin Zeaxanthin, Violacein, Pyocyanin, Xanthomonadin, Rubrolone, Riboflavin, Carotenoids, Indochrome, Porphyrins, Glaukothalin, Flexirubin, and Prodigiosin.

In some embodiments of the disclosure, the at least one photosynthetic organism-derived chromophore is extracted from a photosynthetic organism source including, but not limited to, a marine or terrestrial plant, algae or microorganism. In some embodiments of the disclosure, the at least one xanthene dye is extracted from non-photosynthetic prokaryotic organism including, but not limited to, a bacterium or microorganism. For example, the at least one photosynthetic organism-derived chromophore can be extracted from a pulverized marine, terrestrial plant, or algae or from a cell pellet of algae or a microorganism and the at least one xanthene dye can be extracted from a cell pellet of bacteria using an organic solvent such as acetone, benzene, chloroform, ethyl acetate, ethanol, methanol, petroleum ether, propylene glycol, hexane and DMSO. The at least one photosynthetic organism-derived chromophore or the at least one xanthene dye can then be purified by techniques such as column chromatography (reverse phase or silica gel), liquid chromatography, HPLC, thin layer chromatography (TLC), and gel permeation chromatography. The chromophore containing compositions resulting from the extraction or from the purification can be characterized using techniques such as UV-vis, FTIR, ESI-MS, and NMR.

Xanthene derivatives

Exemplary xanthene dyes that are useful in the compositions, methods, and uses of the disclosure include, but are not limited to, Eosin B, Eosin B (4',5'-dibromo,2',7'-dinitro-fluorescein, dianion), Eosin Y, Eosin Y (2',4',5',7'-tetrabromo-fluorescein, dianion), Eosin (2',4',5',7'-tetrabromo-fluorescein, dianion), Eosin (2',4',5',7'-tetrabromo-fluorescein, dianion) methyl ester, Eosin (2',4',5',7'-tetrabromo-fluorescein, monoanion) p-isopropylbenzyl ester, Eosin derivative (2',7'-dibromo-fluorescein, dianion), Eosin derivative (4',5'-dibromo-fluorescein, dianion), Eosin derivative (2',7'-dichloro-fluorescein, dianion), Eosin derivative (4',5'-dichloro-fluorescein, dianion); Eosin derivative (2',7'-diiodo-fluorescein, dianion), Eosin derivative (4',5'-diiodo-fluorescein, dianion), Eosin derivative (tribromo-fluorescein, dianion), Eosin derivative (2',4',5',7'-tetrachloro-fluorescein, dianion), Eosin; Eosin dicetylpyridinium chloride ion pair, Erythrosin B (2',4',5',7'-tetraiodo-fluorescein, dianion), Erythrosine, Erythrosin dianion, Erythiosin B, Fluorescein, Fluorescein dianion, Phloxin B (2',4',5',7'-tetrabromo-3,4,5,6-tetrachloro-fluorescein, dianion), Phloxin B (tetrachloro- tetrabromo-fluorescein), Phloxine B, Rose Bengal (3,4,5,6-tetrachloro-2',4',5',7'- tetraiodofluorescein, dianion), Pyronin G, Pyronin J, Pyronin Y. Other xanthene dyes that are useful in the compositions, methods, and uses of the disclosure also include, but are not limited to, rhodamine dyes such as 4,5-dibromo-rhodamine methyl ester; 4,5-dibromo- rhodamine n-butyl ester; rhodamine 101 methyl ester; rhodamine 123; rhodamine 6G; rhodamine 6G hexyl ester; tetrabromo-rhodamine 123; and tetramethylrhodamine ethyl ester.

In some embodiments of the disclosure, the xanthene chromophore is selected from Eosin, Eosin Y, Eosin B, Erythrosin B, Fluorescein, Rose Bengal, Phloxin B, or combinations thereof. In some embodiments of the disclosure, the xanthene chromophore is selected from Eosin Y, Eosin B, Erythrosin B, Fluorescein, Rose Bengal, Phloxin B, or combinations thereof. In some embodiments of the disclosure, the xanthene chromophore is Eosin. In some embodiments of the disclosure, the xanthene chromophore is Eosin B. In some embodiments of the disclosure, the xanthene chromophore is Eosin Y. In some embodiments of the disclosure, the xanthene chromophore is Erythrosin B. In some embodiments of the disclosure, the xanthene chromophore is Fluorescein. In some embodiments of the disclosure, the xanthene chromophore is Rose Bengal. In some embodiments of the disclosure, the xanthene chromophore is Phloxin B.

Combining chromophores may increase photo-absorption by the combined dye molecules and enhance absorption and photo-biomodulation selectivity. When such multi-chromophore compositions are illuminated with light, energy transfer can occur between the chromophores. This process, known as resonance energy transfer, is a widely prevalent photophysical process through which an excited'donor' chromophore (also referred to herein as first chromophore) transfers its excitation energy to an'acceptor' chromophore (also referred to herein as second chromophore). The efficiency and directedness of resonance energy transfer depends on the spectral features of donor and acceptor chromophores. In particular, the flow of energy between chromophores is dependent on a spectral overlap reflecting the relative positioning and shapes of the absorption and emission spectra. More specifically, for energy transfer to occur, the emission spectrum of the donor chromophore must overlap with the absorption spectrum of the acceptor chromophore.

Energy transfer manifests itself through decrease or quenching of the donor emission and a reduction of excited state lifetime accompanied also by an increase in acceptor emission intensity. To enhance the energy transfer efficiency, the donor chromophore should have good abilities to absorb photons and emit photons. Furthermore, the more overlap there is between the donor chromophore's emission spectra and the acceptor chromophore's absorption spectra, the better a donor chromophore can transfer energy to the acceptor chromophore.

In certain embodiments, the at least one photosynthetic organism-derived (i.e., the first chromophore) is the donor chromophore and the at least xanthene dye (second chromophore) is the acceptor chromophore. In other embodiments, the at least second chromophore is the donor chromophore and the at least one photosynthetic organism-derived chromophore (i.e., the first chromophore) is the acceptor chromophore.

In some embodiments, the first chromophore has an emission spectrum that overlaps at least about 80%, about 75%, about 70%, about 65%, about 60%, about 55%, about 50%, about 45%, about 40%, about 35%, about 30%, about 25%, about 20%, about 15% or about 10% with an absorption spectrum of the at least second chromophore. In some embodiments the first chromophore has an emission spectrum that overlaps at least about 20% with an absorption spectrum of the at least second chromophore.

In some embodiments, the first chromophore has an emission spectrum that overlaps at least between about 1%-10%, between about 5%-15%, between about 10%-20%, between about 15%-25%, between about 20%-30%, between about 25%-35%, between about 30%-40%, between about 35%-45%, between about 50%-60%, between about 55%-65% or between about 60%-70% with an absorption spectrum of the at least second chromophore.

In other embodiments, the at least second chromophore has an emission spectrum that overlaps at least about 80%, about 75%, about 70%, about 65%, about 60%, about 55%, about 50%, about 45%, about 40%, about 35%, about 30%, about 25%, about 20%, about 15% or about 10% with an absorption spectrum of the first chromophore. In some embodiments, the at least second chromophore has an emission spectrum that overlaps at least about 20% with an absorption spectrum of the first chromophore. In some embodiments, the at least second chromophore has an emission spectrum that overlaps at least between about 1%-10%, between about 5%-15%, between about 10%-20%, between about 15%-25%, between about 20%-30%, between about 25%-35%, between about 30%- 40%, between about 35%-45%, between about 50%-60%, between about 55%-65% or between about 60%-70% with an absorption spectrum of the at least first chromophore.

% spectral overlap, as used herein, means the % overlap of a donor chromophore's emission wavelength range with

an acceptor chromophore's absorption wavelength range, measured at spectral full width quarter maximum (FWQM). For example, if the spectral FWQM of the acceptor chromophore's absorption spectrum is about 60 nm and the overlap of the donor chromophore's spectrum with the absorption spectrum of the acceptor chromophore is about 30 nm, then the % overlap can be calculated as 30 nm / 60 nm x 100 = 50%.

The at least one photosynthetic organism-derived chromophore or the at least one xanthene dye can be present in an amount of about 0.0001%-40% by weight of the biophotonic composition, such as 0.0001%-40% by weight of the biophotonic composition. When present, a third chromophore can be present in an amount of about 0.0001%-40% by weight of the biophotonic composition, such as 0.0001%-40% by weight of the biophotonic composition. In certain embodiments, the first chromophore is present in an amount of about 0.0001%-2%, about 0.001%-3%, about 0.001%-0.01%, about 0.005%-0.1%, about 0.1%- 0.5%, about 0.5%-2%, about 1%-5%, about 2.5%-7.5%, about 5%-10%, about 7.5%-12.5%, about 10%-15%, about 12.5%-17.5%, about 15%-20%, about 17.5%-22.5%, about 20%-25%, about 22.5%-27.5%, about 25%-30%, about 27.5%-32.5%, about 30%-35%, about 32.5%- 37.5%, or about 35%-40% by weight of the biophotonic composition. In some embodiments, the first chromophore is present in an amount of 0.0001%-2%, 0.001%-3%, 0.001%-0.01%, 0.005%-0.1%, 0.1%-0.5%, 0.5%-2%, 1%-5%, 2.5%-7.5%, 5%-10%, 7.5%-12.5%, 10%-15%, 12.5%-17.5%, 15%-20%, 17.5%-22.5%, 20%-25%, 22.5%-27.5%, 25%-30%, 27.5%-32.5%, 30%-35%, 32.5%-37.5%, or 35%-40% by weight of the biophotonic composition. In certain embodiments, the at least second chromophore is present in an amount of about 0.0001%-2%, about 0.001%-3%, about 0.001%-0.01%, about 0.005%-0.1%, about 0.1%-0.5%, about 0.5%- 2%, about 1%-5%, about 2.5%-7.5%, about 5%-10%, about 7.5%-12.5%, about 10%-15%, about 12.5%-17.5%, about 15%-20%, about 17.5%-22.5%, about 20%-25%, about 22.5%- 27.5%, about 25%-30%, about 27.5%-32.5%, about 30%-35%, about 32.5%-37.5%, or about 35%-40% by weight of the biophotonic composition. In some embodiments, the at least second chromophore is present in an amount of 0.0001%-2%, 0.001%-3%, 0.001%-0.01%, 0.005%-0.1%, 0.1%-0.5%, 0.5%-2%, 1%-5%, 2.5%-7.5%, 5%-10%, 7.5%-12.5%, 10%-15%, 12.5%-17.5%, 15%-20%, 17.5%-22.5%, 20%-25%, 22.5%-27.5%, 25%-30%, 27.5%-32.5%, 30%-35%, 32.5%-37.5%, or 35%-40% by weight of the biophotonic composition. In certain embodiments, a third chromophore is present in an amount of about 0.0001%-2%, about 0.001%-3%, about 0.001%-0.01%, about 0.005%-0.1%, about 0.1%-0.5%, about 0.5%-2%, about 1%-5%, about 2.5%-7.5%, about 5%-10%, about 7.5%-12.5%, about 10%-15%, about 12.5%-17.5%, about 15%-20%, about 17.5%-22.5%, about 20%-25%, about 22.5%-27.5%, about 25%-30%, about 27.5%-32.5%, about 30%-35%, about 32.5%-37.5%, or about 35%- 40% by weight of the biophotonic composition. In certain embodiments, a third chromophore is present in an amount of 0.0001%-2%, 0.001%-3%, 0.001%-0.01%, 0.005%- 0.1%, 0.1%-0.5%, 0.5%-2%, 1%-5%, 2.5%-7.5%, 5%-10%, 7.5%-12.5%, 10%-15%, 12.5%- 17.5%, 15%-20%, 17.5%-22.5%, 20%-25%, 22.5%-27.5%, 25%-30%, 27.5%-32.5%, 30%- 35%, 32.5%-37.5%, or 35%-40% by weight of the biophotonic composition. In certain embodiments, the total weight of combination of chromophores may be in the amount of about 0.005%-1%, about 0.05%-2%, about 1%-5%, about 2.5%-7.5%, about 5%-10%, about 7.5%-12.5%, about 10%-15%, about 12.5%-17.5%, about 15%-20%, about 17.5%-22.5%, about 20%-25%, about 22.5%-27.5%, about 25%-30%, about 27.5%-32.5%, about 30%-35%, about 32.5%-37.5%, or about 35%-40.0% by weight of the biophotonic composition. In some embodiments, the total weight of combination of chromophores may be in the amount of 0.0001%-2%, 0.005%-1%, 0.05%-2%, 1%-5%, 2.5%-7.5%, 5%-10%, 7.5%-12.5%, 10%-15%, 12.5%-17.5%, 15%-20%, 17.5%-22.5%, 20%-25%, 22.5%-27.5%, 25%-30%, 27.5%-32.5%, 30%-35%, 32.5%-37.5%, or 35%-40.0% by weight of the biophotonic composition. In certain embodiments, the total weight of combination of chromophores may be in the amount of about 0.005%-1% by weight of the biophotonic composition, such as 0.005%-1% by weight of the biophotonic composition. In certain embodiments, the total weight of combination of chromophores may be in the amount of about 0.05%-2% by weight of the biophotonic composition, such as 0.05%-2% by weight of the biophotonic composition. In certain embodiments, the total weight of combination of chromophores may be in the amount of about 1%-5% by weight of the biophotonic composition, such as 1%-5% by weight of the biophotonic composition. In certain embodiments, the total weight of combination of chromophores may be in the amount of about 2.5%-7.5% by weight of the biophotonic composition, such as 2.5%-7.5% by weight of the biophotonic composition. In certain embodiments, the total weight of combination of chromophores may be in the amount of about 5%-10% by weight of the biophotonic composition, such as 5%-10% by weight of the biophotonic composition. The concentration of the chromophores to be used can be selected based on the desired intensity and duration of the biophotonic activity from the biophotonic composition, and on the desired medical or cosmetic effect. For example, some dyes such as xanthene dyes reach a saturation concentration' after which further increases in concentration do not provide substantially higher emitted fluorescence. Further increasing the chromophores concentration above the saturation concentration can reduce the amount of activating light passing through the matrix. Therefore, if more fluorescence is required for a certain application than activating light, a high concentration of chromophores can be used. However, if a balance is required between the emitted fluorescence and the activating light, a concentration close to or lower than the saturation concentration can be chosen.

Suitable additional chromophores (synthetic or derived from natural source) that may be included in the biophotonic compositions of the present disclosure include, but are not limited to the following:

*Chlorophyll dyes*

Exemplary chlorophyll dyes that are useful in the compositions, methods, and uses of the disclosure, include but are not limited to chlorophyll a, chlorophyll b, oil soluble chlorophyll, bacteriochlorophyll a, bacteriochlorophyll b, bacteriochlorophyll c, bacteriochlorophyll d, protochlorophyll, protochlorophyll a, amphiphilic chlorophyll derivative 1, and amphiphilic chlorophyll derivative 2.

*Methylene blue dyes*

Exemplary methylene blue derivatives that are useful in the compositions, methods, and uses of the disclosure include, but are not limited to, 1-methyl methylene blue; 1,9-dimethyl methylene blue; methylene blue; methylene blue (16 μM); methylene blue (14 μM); methylene violet; bromomethylene violet; 4-iodomethylene violet; 1,9-dimethyl-3-dimethyl- amino-7-diethyl-amino-phenothiazine; and 1,9-dimethyl-3-diethylamino-7-dibutyl-amino- phenothiazine.

*Azo dyes*

Exemplary azo (or diazo-) dyes that are useful in the compositions, methods, and uses of the disclosure include, but are not limited to, methyl violet, neutral red, para red (pigment red 1), amaranth (Azorubine S), Carmoisine (azorubine, food red 3, acid red 14), allura red AC (FD&C 40), tartrazine (FD&C Yellow 5), orange G (acid orange 10), Ponceau 4R (food red 7), methyl red (acid red 2), and murexide-ammonium purpurate. In some embodiments of the disclosure, the one or more chromophores that are useful in the compositions, methods, and uses of the disclosure include, but are not limited to, Acid black 1, Acid blue 22, Acid blue 93, Acid fuchsin, Acid green, Acid green 1, Acid green 5, Acid magenta, Acid orange 10, Acid red 26, Acid red 29, Acid red 44, Acid red 51, Acid red 66, Acid red 87, Acid red 91, Acid red 92, Acid red 94, Acid red 101, Acid red 103, Acid roseine, Acid rubin, Acid violet 19, Acid yellow 1, Acid yellow 9, Acid yellow 23, Acid yellow 24, Acid yellow 36, Acid yellow 73, Acid yellow S, Acridine orange, Acriflavine, Alcian blue, Alcian yellow, Alcohol soluble eosin, Alizarin, Alizarin blue 2RC, Alizarin carmine, Alizarin cyanin BBS, Alizarol cyanin R, Alizarin red S, Alizarin purpurin, Aluminon, Amido black 10B, Amidoschwarz, Aniline blue WS, Anthracene blue SWR, Auramine O, Azocannine B, Azocarmine G, Azoic diazo 5, Azoic diazo 48, Azure A, Azure B, Azure C, Basic blue 8, Basic blue 9, Basic blue 12, Basic blue 15, Basic blue 17, Basic blue 20, Basic blue 26, Basic brown 1, Basic fuchsin, Basic green 4, Basic orange 14, Basic red 2 (Saffranin O), Basic red 5, Basic red 9, Basic violet 2, Basic violet 3, Basic violet 4, Basic violet 10, Basic violet 14, Basic yellow 1, Basic yellow 2, Biebrich scarlet, Bismarck brown Y, Brilliant crystal scarlet 6R, Calcium red, Carmine, Carminic acid (acid red 4), Celestine blue B, China blue, Cochineal, Celestine blue, Chrome violet CG, Chromotrope 2R, Chromoxane cyanin R, Congo corinth, Congo red. Cotton blue, Cotton red, Croceine scarlet, Crocin, Crystal ponceau 6R, Crystal violet, Dahlia, Diamond green B, DiOC6, Direct blue 14, Direct blue 58, Direct red, Direct red 10, Direct red 28, Direct red 80, Direct yellow 7, Eosin B, Eosin Bluish, Eosin, Eosin Y, Eosin yellowish, Eosinol, Erie garnet B, Eriochrome cyanin R, Erythrosin B, Ethyl eosin, Ethyl green, Ethyl violet, Evans blue, Fast blue B, Fast green FCF, Fast red B, Fast yellow, Fluorescein, Food green 3, Gallein, Gallamine blue, Gallocyanin, Gentian violet, Haematein, Haematine, Haematoxylin, Helio fast rubin BBL, Helvetia blue, Hematein, Hematine, Hematoxylin, Floffman's violet, Imperial red, Indocyanin green, Ingrain blue, Ingrain blue 1, Ingrain yellow 1, INT, Kermes, Kermesic acid, Kemechtrot, Lac, Laccaic acid, Lauth's violet, Light green, Lissamine green SF, Luxol fast blue, Magenta 0, Magenta I, Magenta II, Magenta III, Malachite green, Manchester brown, Martius yellow, Merbromin, Mercurochrome, Metanil yellow, Methylene azure A, Methylene azure B, Methylene azure C, Methylene blue, Methyl blue, Methyl green, Methyl violet, Methyl violet 2B, Methyl violet 10B, Mordant blue 3, Mordant blue 10, Mordant blue 14, Mordant blue 23, Mordant blue 32, Mordant blue 45, Mordant red 3, Mordant red 11, Mordant violet 25, Mordant violet 39 Naphthol blue black, Naphthol green B, Naphthol yellow S, Natural black 1, Natural red, Natural red 3, Natural red 4, Natural red 8, Natural red 16, Natural red 25, Natural red 28, Natural yellow 6, NBT, Neutral red, New fuchsin, Niagara blue 3B, Night blue, Nile blue, Nile blue A, Nile blue oxazone, Nile blue sulphate, Nile red, Nitro BT, Nitro blue tetrazolium, Nuclear fast red, Oil red O, Orange G, Orcein, Pararosanilin, Phloxine B, phycobilins, Phycocyanins, Phycoerythrins. Phycoerythrincyanin (PEC), Phthalocyanines, Picric acid, Ponceau 2R, Ponceau 6R, Ponceau B, Ponceau de Xylidine, Ponceau S, Primula, Purpurin, Pyronin B, Pyronin G, Pyronin Y, Rhodamine B, Rosanilin, Rose bengal, Saffron, Safranin O, Scarlet R, Scarlet red, Scharlach R, Shellac, Sirius red F3B, Solochrome cyanin R, Soluble blue, Solvent black 3, Solvent blue 38, Solvent red 23, Solvent red 24, Solvent red 27, Solvent red 45, Solvent yellow 94, Spirit soluble eosin, Sudan III, Sudan IV, Sudan black B, Sulfur yellow S, Swiss blue, Tartrazine, Thioflavine S, Thioflavine T, Thionin, Toluidine blue, Toluyline red, Tropaeolin G, Trypaflavine, Trypan blue, Uranin, Victoria blue 4R, Victoria blue B, Victoria green B, Water blue I, Water soluble eosin, Xylidine ponceau, or Yellowish eosin.

In certain embodiments, the biophotonic composition of the present disclosure includes any of the chromophores listed above, or a combination thereof, so as to provide a synergistic biophotonic effect at the application site.

Without being bound to any particular theory, a synergistic effect of the chromophore combinations means that

the biophotonic effect is greater than the sum of their individual effects. Advantageously, this may translate to increased reactivity of the biophotonic composition, faster or improved treatment time. Also, the treatment conditions need not be altered to achieve the same or better treatment results, such as time of exposure to light, power of light source used, and wavelength of light used. In other words, use of synergistic combinations of chromophores may allow the same or better treatment without necessitating a longer time of exposure to a light source, a higher power light source or a light source with different wavelengths.

By means of synergistic effects of the chromophore combinations in the composition, chromophores which cannot normally be activated by an activating light (such as a blue light from an LED) can be activated through energy transfer from chromophores which are activated by the activating light. In this way, the different properties of photoactivated chromophores can be harnessed and tailored according to the cosmetic or the medical therapy required.

In some embodiments, the chromophore or chromophores are selected such that their emitted fluorescent light, on photoactivation, is within one or more of the green, yellow, orange, red and infrared portions of the electromagnetic spectrum, for example having a peak wavelength within the range of about 490 nm to about 800 nm. In certain embodiments, the emitted fluorescent light has a power density of between about 0.005 $mW/cm^2$ to about 10 $mW/cm^2$, about 0.5 $mW/cm^2$ to about 5 $mW/cm^2$.

(b) Oxidants

In some embodiments, the biophotonic compositions, methods, and uses of the present disclosure comprise one or more oxidants as a source of oxygen radicals or singlet oxygen. Peroxide compounds are oxidants that contain the peroxy group (R-O-O-R), which is a chainlike structure containing two oxygen atoms, each of which is bonded to the other and a radical or some element. When a biophotonic composition of the present disclosure comprising an oxidant is illuminated with light, the chromophores are excited to a higher energy state. When the chromophores' electrons return to a lower energy state, they emit photons with a lower energy level, thus causing the emission of light of a longer wavelength (Stokes' shift). In the proper environment, some of this energy is transferred to oxygen or the reactive hydrogen peroxide and causes the formation of oxygen radicals, such as singlet oxygen. The singlet oxygen and other reactive oxygen species generated by the activation of the biophotonic composition are thought to operate in a hormetic fashion. That is, a health beneficial effect that is brought about by the low exposure to a normally toxic stimuli (e.g. reactive oxygen), by stimulating and modulating stress response pathways in cells of the targeted tissues. Endogenous response to exogenous generated free radicals (reactive oxygen species) is modulated in increased defense capacity against the exogenous free radicals and induces acceleration of healing and regenerative processes. Furthermore, the extreme sensitivity of bacteria to exposure to free radicals makes the biophotonic composition of the present disclosure potentially a bactericidal composition.

Peroxide compounds are oxidants that contain the peroxy group (R-O-O-R), which is a chainlike structure containing two oxygen atoms, each of which is bonded to the other and a radical or some element. Suitable oxidants for preparation of the active medium include, but are not limited to:

Hydrogen peroxide ($H_2O_2$) is a powerful oxidizing agent, and breaks down into water and oxygen and does not form any persistent, toxic residual compound. A suitable range of concentration over which hydrogen peroxide can be used in the biophotonic composition is from about 0.01% to about 30%, about 1% to about 25%, about 5% to about 20%, about 10% to about 15%, or less than about 20% by weight of the total composition. In some embodiments, hydrogen peroxide is present in an amount from about 0.1% to about 12%, from about 1% to about 12%, from about 3.5% to about 12%, from about 3.5% to about 6% or from about 0.1% to about 6% by weight of the total composition. In some embodiments, hydrogen peroxide is present in an amount from 0.01% to 30%, 1% to 25%, 5% to 20%, 10% to 15%, or less than 20% by weight of the total composition. In some embodiments, hydrogen peroxide is present in an amount from 0. 1% to 12%, from 1% to 12%, from 3.5% to 12%, from 3.5% to 6% or from 0.1% to 6% by weight of the total composition.

Urea hydrogen peroxide (also known as urea peroxide, carbamide peroxide or percarbamide) is soluble in water and contains approximately 35% hydrogen peroxide. Urea peroxide brakes down to urea and hydrogen peroxide in a slow-release fashion that can be accelerated with heat or photochemical reactions. The released urea ($(NH_2)_2CO_2$), is highly soluble in water and is a powerful protein denaturant. It increases solubility of some proteins and enhances rehydration of the skin and/or mucosa. A suitable range of concentration over which urea peroxide can be used in the biophotonic composition of the present disclosure is less than about 25%, or less than about 20%, or less than about 15%, or less than about 10%, or less than about 5%, or from about 0.1% to about 5%, or from about 1% to about 15% by weight of the total composition. In some embodiments, urea peroxide is present in less than 25%, or less than 20%, or less than 15%, or less than 10%, or less than 5%, or from 0.1 to 5%, or from 1% to 15% by weight of the total composition. In some embodiments, urea peroxide is present in an amount from about 0.3% to about 36%, from about 3% to about 36%, or from about 10% to about 36%, or from about 3% to about 16% or from about 0.3% to about 16% by weight of the total composition. In some embodiments, urea peroxide is present in an amount from 0.3% to 36%), from 3% to 36%, or from 10% to 36%, or from 3% to 16% or from 0.3% to 16% by weight of the total composition. In some embodiments,

urea peroxide is present in an amount of about 2% by weight of the total composition, such as 2%) by weight of the total composition. In some embodiments, urea peroxide is present in an amount of about 3% by weight of the total composition, such as 3% by weight of the total composition. In some embodiments, urea peroxide is present in an amount of about 6% by weight of the total composition, such as 6% by weight of the total composition. In some embodiments, urea peroxide is present in an amount of about 8% by weight of the total composition, such as 8% by weight of the total composition. In some embodiments, urea peroxide is present in an amount of about 12% by weight of the total composition, such as 12% by weight of the total composition.

Benzoyl peroxide consists of two benzoyl groups (benzoic acid with the H of the carboxylic acid removed) joined by a peroxide group. It is found in treatments for acne, in concentrations varying from 2.5% to 10%. The released peroxide groups are effective at killing bacteria. Benzoyl peroxide also promotes skin turnover and clearing of pores, which further contributes to decreasing bacterial counts and reduce acne. Benzoyl peroxide breaks down to benzoic acid and oxygen upon contact with skin, neither of which is toxic. A suitable range of concentration over which benzoyl peroxide can be used in the biophotonic composition is from about 2.5% to about 20%, or about 2.5% to about 10% by weight of the total composition. In some embodiments, benzoyl peroxide is present in an amount from 2.5% to 20%, or 2.5% to 10% by weight of the total composition. In some embodiments, benzoyl peroxide is present in an amount from about 1% to about 10%, or from about 1% to about 8%, or from about 2.5% to about 5% by weight of the total composition. In some embodiments, benzoyl peroxide is present in an amount from 1% to 10%, or from 1% to 8%, or from 2.5% to 5% by weight of the total composition.

In some embodiments, the peroxide or peroxide precursor is a peroxy acid, an alkali metal peroxide, an alkali metal percarbonate, a peroxyacetic acid, an alkali metal perborate, or methyl ethyl ketone peroxide. In some embodiments, the oxidant is methyl ethyl ketone peroxide. A suitable range of concentration over which methyl ethyl ketone peroxide can be used in the biophotonic composition is from about 0.01%) to about 15% by weight of the total composition, such as 0.01% to 15% by weight of the total composition.

(c) Carrier Medium

In some embodiments, the biophotonic compositions, methods, and uses of the present disclosure comprise a carrier medium made from one or more thickening agents. Thickening agents are present in an amount and ratio sufficient to provide a desired viscosity, flexibility, rigidity, tensile strength, tear strength, elasticity, and adhesiveness. The thickening agents are selected so that the chromophore(s) can remain photoactive in the carrier medium. The thickening agents are also selected according to the optical transparency of the carrier medium. The carrier medium should be able to transmit sufficient light to activate the at least one chromophore and, in embodiments where fluorescence is emitted by the activated chromophore, the carrier medium should also be able to transmit the emitted fluorescent light to tissues. It will be recognized by persons skilled in the art that the thickening agent is an appropriate medium for the chromophore(s) selected. For example, the inventors have noted that some xanthene dyes do not fluoresce in non-hydrated media, so hydrated polymers or polar solvents may be used. The thickening agents should also be selected according to the intended use. For example, if the biophotonic composition is to be applied onto tissue, the carrier medium is preferably biocompatible, or the carrier medium has an outside layer of a biocompatible composition which will interface the tissue.

*Thickening agents*

In some embodiments, the content of a thickening agent is present in the composition in an amount of from about 0.001 % to about 40 % (w/w %) of the total weight. In certain embodiments, the total content of the thickening agent is about 0.001%-0.01%, about 0.005%-0.05%, about 0.01%-0.1, about 0.05%-0.5%, about 0.1%-1%, about 0.5%-5%, about 1%-5%, about 2.5%-7.5%, about 5%-10%, about 7.5%-12.5%, about 10%-15%, about 12.5%-17.5%, about 15%-20%, about 15%-25%, about 20%-30%, about 25%-35%, or about 30%-40% by weight of the total composition. In some embodiments, the total content of the thickening agent is 0.001%-0.01%, 0.005%-0.05%, 0.01%-0.1, 0.05%-0.5%, 0.1%-1%, 0.5%-5%, 1%-5%, 2.5%-7.5%, 5%-10%, 7.5%-12.5%, 10%-15%, 12.5%-17.5%, 15%-20%, 15%-25%, 20%-30%, 25%-35%, or 30%-40% by weight of the total composition. It will be recognized by one of skill in the art that the viscosity, flexibility, rigidity, tensile strength, tear strength, elasticity, and adhesiveness can be adjusted by varying the content of the thickening agent. Methods of determining viscosity, flexibility, rigidity, tensile strength, tear strength, elasticity, and adhesiveness are known in the art.

Thickening agents that can be used to prepare the biophotonic compositions of the present disclosure include but are not limited to a hydrophilic polymer, a hygroscopic polymer or a hydrated polymer. The thickening agent may be polyanionic in charge character. The thickening agent may comprise carboxylic functional groups, and may further contain 2 to 7 carbon atoms per functional group. The thickening agents may include polymers, copolymers, and monomers of: vinylpyrrolidones, methacrylamides, acrylamides N-vinylimidazoles, carboxy vinyls, vinyl esters, vinyl ethers,

silicones, polyethyleneoxides, polyethyleneglycols, vinylalcohols, sodium acrylates, acrylates, maleic acids, N,N-dimethylacrylamides, diacetone acrylamides, acrylamides, acryloyl morpholine, pluronic, collagens, polyacrylamides, polyacrylates, polyvinyl alcohols, polyvinylenes, polyvinyl silicates, polyacrylates substituted with a sugar (e.g., sucrose, glucose, glucosamines, galactose, trehalose, mannose, or lactose), acylamidopropane sulfonic acids, tetramethoxyorthosilicates, methyltrimethoxyorthosilicates, tetraalkoxyorthosilicates, trialkoxyorthosilicates, glycols, propylene glycol, glycerine, polysaccharides, alginates, dextrans, cyclodextrin, celluloses, modified celluloses, oxidized celluloses, chitosans, chitins, guars, carrageenans, hyaluronic acids, inulin, starches, modified starches, agarose, methylcelluloses, plant gums, hyaluronans, hydrogels, gelatins, glycosaminoglycans, carboxymethyl celluloses, hydroxyethyl celluloses, hydroxy propyl methyl celluloses, pectins, low-methoxy pectins, cross-linked dextrans, starch-acrylonitrile graft copolymers, starch sodium polyacrylate, hydroxyethyl methacrylates, hydroxyl ethyl acrylates, polyvinylene, polyethylvinylethers, polymethyl methacrylates, polystyrenes, polyurethanes, polyalkanoates, polylactic acids, polylactates, poly(3-hydroxybutyrate), sulfonated hydrogels, AMPS (2-aciylamido-2-methyl-1-propanesulfbnic acid), SEM (sulfoethylmethacrylate), SPM (sulfopropyl methacrylate), SPA (sulfopropyl aciylate), N,N-dimethyl-N-methacryloxyethyl-N-(3-sulfopropyl)ammonium betaine, methacryllic acid amidopropyl-dimethyl ammonium sulfobetaine, SPI (itaconic acid-bis(1-propylsulfonizacid-3)ester di-potassium salt), itaconic acids, AMBC (3-acrylamido-3-methylbutanoic acid), beta-carboxyethyl acrylate (acrylic acid dimers), and maleic anhydride-methylvinyl ether polymers, derivatives thereof, salts thereof, acids thereof, and combinations thereof In some embodiments, the thickening agent comprises 2-Hydroxyethyl methacrylate (HEMA) either alone or in addition to another thickening agent. In some embodiments, the 2-Hydroxyethyl methacrylate (HEMA) is added in a form such as microspheres or in a further physically reduced form such as a finely ground particulate form or in a pulverized, powder form.

In certain embodiments, the at least one thickening agent is a synthetic polymer selected from one or more of vinyl polymers, polyoxythylene-polyoxypropylene copolymers, poly(ethylene oxide), acrylamide polymers and derivatives and salts thereof. In a further embodiment the vinyl polymer is selected from one or more of polyacrylic acid, polymethacrylic acid, polyvinyl pyrrolidone and polyvinyl alcohol. In other embodiments, the vinyl polymer is a carboxy vinyl polymer or a carbomer obtained by the polymerization of acrylic acid. The carboxy vinyl polymer or carbomer may be cross-linked.

As mentioned above, in some embodiments, the at least one thickening agent of the carrier medium is one or more carbomers. Carbomers are synthetic high molecular weight polymers of acrylic acid that are crosslinked with either allylsucrose or allylethers of pentaerythritol having a molecular weight of about $3 \times 10^6$. The gelation mechanism depends on neutralization of the carboxylic acid moiety to form a soluble salt. The polymer is hydrophilic and produces sparkling clear gels when neutralized. Carbomers are available as fine white powders which disperse in water to form acidic colloidal suspensions (a 1% dispersion has approximately pH 3) of low viscosity. Neutralization of these suspensions using a base, for example sodium, potassium or ammonium hydroxides, low molecular weight amines and alkanolamines, results in the formation of clear translucent gels.

In some embodiments, the carbomer is a Carbopol®. Such polymers are commercially available from B.F. Goodrich or Lubrizol under the designation Carbopol® 71G NF, 420, 430, 475, 488, 493, 910, 934, 934P, 940, 971PNF, 974P NF, 980 NF, 981 NF and the like. Carbopols are versatile controlled-release polymers, as described by Brock (Pharmacotherapy, 14:430-7 (1994), incorporated herein by reference) and Durrani (Pharmaceutical Res. (Supp.) 8:S-135 (1991), incorporated herein by reference), and belong to a family of carbomers which are synthetic, high molecular weight, non-linear polymers of acrylic acid, cross-linked with polyalkenyl polyether. In certain embodiments, the carbomer is Carbopol® 940, Carbopol® 980, ETD 2020NF, Carbopol® 1382, 71G NF, 971P NF, 974P NF, 980 NF, 981 NF, 5984 EP, ETF 2020 NF, Ultrez 10 NF, Ultrez 20, Ultrez 21, 1342 NF, 934 NF, 934P NF, 940 NF or 941 NF, or combinations thereof. In some embodiments, the carbomer is cross-linked with alkyl acrylate or allyl pentaerythritol. In some embodiments, the carbomer is present in the composition in an amount of from about 0.01 wt% to about 15 wt%, or about 0.05 wt% to about 5 wt%, or about 0.5 wt% to about 2 wt%. In some embodiments, the carbomer is present in the composition in an amount of from 0.01 wt% to 15 wt%, or 0.05 wt% to 5 wt%, or 0.5 wt% to 2 wt%.

In certain embodiments, the at least one thickening agent of the carrier medium is a glycol, such as ethylene glycol or propylene glycol. In further embodiments, the at least one thickening agent of the carrier medium is a poly (ethylene oxide) polymer (such as POLYOX from Dow Chemical), linear PVP and cross-linked PVP, PEG/PPG copolymers (such as BASF Pluracare L1220), ethylene oxide (EO)-propylene oxide (PO) block copolymers (such as polymers sold under the trade mark Pluronic available from BASF Corporation), ester gum, shellac, pressure sensitive silicone adhesives (such as BioPSA from Dow-Corning), or mixtures thereof. In some embodiments, a copolymer comprises (PVM/MA). In some embodiments, a copolymer comprises poly(methylvinylether/maleic anhydride). In some embodiments, a copolymer comprises poly (methylvinylether/maleic acid). In some embodiments, a copolymer comprises poly (methylvinylether/maleic acid) half esters. In some embodiments, a copolymer comprises poly (methylvinylether/maleic acid) mixed salts.

In certain embodiments of the disclosure, the at least one thickening agent of the carrier medium is a protein-based polymer. Such protein-based polymer may be selected from gelatin ,collagen, or a combination thereof. For

example, the composition may comprise at least about 4 wt%, about 4 wt% to about 25 wt%, or about 10 wt% to about 20 wt% gelatin within the biophotonic composition. In some embodiments, the composition may comprise at least 4 wt%, 4 wt% to 25 wt%, or 10 wt% to 20 wt% gelatin within the biophotonic composition. The composition may comprise at least about 5 wt%, about 5 wt% to about 25 wt%, or about 10 wt% to about 20 wt% collagen within the biophotonic composition. In some embodiments, the composition may comprise at least 5 wt%, 5 wt% to 25 wt%, or 10 wt% to 20 wt% collagen within the biophotonic composition. Alternatively, a lower weight percentage of protein-based polymers may be used together with chemical cross-linkers or any other cross-linking means.

In certain embodiments of the disclosure, the at least one thickening agent of the carrier medium is sodium hyaluronate. For example, the composition may comprise at least about 4 wt%, about 4 wt% to about 25 wt%, or about 10 wt% to about 20 wt% sodium hyaluronate within the biophotonic composition. In some embodiments, the composition may comprise at least 4 wt%, 4 wt% to 25 wt%, or 10 wt% to 20 wt% sodium hyaluronate within the biophotonic composition.

In certain embodiments of the disclosure, the at least one thickening agent of the carrier medium is a polysaccharide, which may be from at least one of starch, chitosan, chitin, agar, alginates, xanthan, carrageenan, guar gum, gellan gum, pectin, or locust bean gum.

The biophotonic composition of the present disclosure may optionally be provided with a water-insoluble substrate. By "water insoluble", it is meant that the substrate does not dissolve in or readily break apart upon immersion in water. In some embodiments, the water-insoluble substrate is the implement or vehicle for delivering the treatment composition to the skin or target tissue. A wide variety of substances can be used as the water-insoluble substrate. One or more of the following non-limiting characteristics may be desirable: (i) sufficient wet strength for use, (ii) sufficient softness, (iii) sufficient thickness, (iv) appropriate size, (v) air permeability, and (vi) hydrophilicity.

Non-limiting examples of suitable water-insoluble substrates which meet the above criteria include nonwoven substrates, woven substrates, hydroentangled substrates, air entangled substrates, natural sponges, synthetic sponges, polymeric netted meshes, and the like. Some embodiments employ nonwoven substrates since they are economical and readily available.

By "nonwoven", it is meant that the layer is comprised of fibers which are not woven into a fabric but rather are formed into a sheet, mat, or pad layer.

(d) Antimicrobials

According to some embodiments, the biophotonic compositions of the methods and uses of the present disclosure may optionally further comprise one or more antimicrobials. Antimicrobials kill microbes or inhibit their growth or accumulation. Exemplary antimicrobials (or antimicrobial agent) are recited in U.S. Patent Application Publication Nos. 20040009227 and 20110081530.

Suitable antimicrobials for use in the methods of the present disclosure include, but not limited to, phenolic and chlorinated phenolic and chlorinated phenolic compounds, resorcinol and its derivatives, bisphenolic compounds, benzoic esters (parabens), halogenated carbonilides, polymeric antimicrobial agents, thazolines, trichloromethylthioimides, natural antimicrobial agents (also referred to as "natural essential oils"), metal salts, and broad-spectrum antibiotics.

Additionally, the biophotonic composition of the present disclosure comprises a peroxide or peroxide derivative, which upon illumination of the biophotonic composition will lead to the generation oxygen radicals. The extreme sensitivity of bacteria to exposure to free radicals makes the biophotonic composition of the present disclosure potentially a bactericidal composition.

Examples of phenolic and chlorinated phenolic antimicrobial agents that can be used in the compositions defined herein include, but are not limited to: phenol; 2-methyl phenol; 3-methyl phenol; 4-methyl phenol; 4-ethyl phenol; 2,4-dimethyl phenol; 2,5-dimethyl phenol; 3,4-dimethyl phenol; 2,6-dimethyl phenol; 4-n-propyl phenol; 4-n-butyl phenol; 4-n-amyl phenol; 4-tert-amyl phenol; 4-n-hexyl phenol; 4-n-heptyl phenol; mono- and poly-alkyl and aromatic halophenols; p-chlorophenyl; methyl p-chlorophenol; ethyl p-chlorophenol; n-propyl p-chlorophenol; n-butyl p-chlorophenol; n-amyl p-chlorophenol; sec-amyl p-chlorophenol; n-hexyl p-chlorophenol; cyclohexyl p-chlorophenol; n-heptyl p-chlorophenol; n-octyl; p-chlorophenol; o-chlorophenol; methyl o-chlorophenol; ethyl o-chlorophenol; n-propyl o-chlorophenol; n-butyl o-chlorophenol; n-amyl o-chlorophenol; tert-amyl o-chlorophenol; n-hexyl o-chlorophenol; n-heptyl o-chlorophenol; o-benzyl p-chlorophenol; o-benxyl-m-methyl p-chlorophenol; o-benzyl-m,m-dimethyl p-chlorophenol; o-phenylethyl p-chlorophenol; o-phenylethyl-m-methyl p-chlorophenol; 3-methyl p-chlorophenol 3,5-dimethyl p-chlorophenol, 6-ethyl-3-methyl p-chlorophenol, 6-n-propyl-3-methyl p-chlorophenol; 6-iso-propyl-3-methyl p-chlorophenol; 2-ethyl-3,5-dimethyl p-chlorophenol; 6-sec-butyl-3-methyl p-chlorophenol; 2-iso-propyl-3,5-dimethyl p-chlorophenol; 6-diethylmethyl-3-methyl p-chlorophenol; 6-iso-propyl-2-ethyl-3-methyl p-chlorophenol; 2-sec-amyl-3,5-dimethyl p-chlorophenol; 2-diethylmethyl-3,5-dimethyl p-chlorophenol; 6-sec-octyl-3-methyl p-chlorophenol; p-chloro-m-cresol p-bromophenol; methyl p-bromophenol; ethyl p-bromophenol; n-propyl p-bromophenol; n-butyl p-bromophenol; n-amyl p-bromophenol; sec-amyl p-bromophenol; n-hexyl p-bromophenol; cyclohexyl p-bromophenol; o-bromophenol; tert-amyl o-bromophenol; n-hexyl

o-bromophenol; n-propyl-m,m-dimethyl o-bromophenol; 2-phenyl phenol; 4-chloro-2-methyl phenol; 4-chloro-3-methyl phenol; 4-chloro-3,5-dimethyl phenol; 2,4-dichloro-3,5-dimethylphenol; 3,4,5,6-tetabromo-2-methylphenol; 5-methyl-2-pentylphenol; 4-isopropyl-3-methylphenol; para-chloro-metaxylenol (PCMX); chlorothymol; phenoxyethanol; phenoxyisopropanol; and 5-chloro-2-hydroxydiphenylmethane.

Resorcinol and its derivatives can also be used as antimicrobial agents. Examples of resorcinol derivatives include, but are not limited to: methyl resorcinol; ethyl resorcinol; n-propyl resorcinol; n-butyl resorcinol; n-amyl resorcinol; n-hexyl resorcinol; n-heptyl resorcinol; n-octyl resorcinol; n-nonyl resorcinol; phenyl resorcinol; benzyl resorcinol; phenylethyl resorcinol; phenylpropyl resorcinol; p-chlorobenzyl resorcinol; 5-chloro-2,4-dihydroxydiphenyl methane; 4'-chloro-2,4-dihydroxydiphenyl methane; 5-bromo-2,4-dihydroxydiphenyl methane; and 4'-bromo-2,4-dihydroxydiphenyl methane.

Examples of bisphenolic antimicrobial agents that can be used in the compositions defined herein include, but are not limited to: 2,2'-methylene bis-(4-chlorophenol); 2,4,4'trichloro-2'-hydroxy-diphenyl ether, which is sold by Ciba Geigy, Florham Park, N.J. under the tradename Triclosan®; 2,2'-methylene bis-(3,4,6-trichlorophenol); 2,2'-methylene bis-(4-chloro-6-bromophenol); bis-(2-hydroxy-3,5-dichlorophenyl) sulphide; and bis-(2-hydroxy-5-chlorobenzyl)sulphide.

Examples of benzoic esters (parabens) that can be used in the compositions defined herein include, but are not limited to: methylparaben; propylparaben; butylparaben; ethylparaben; isopropylparaben; isobutylparaben; benzylparaben; sodium methylparaben; and sodium propylparaben.

Examples of halogenated carbanilides that can be used in the compositions defined herein include, but are not limited to: 3,4,4'-trichlorocarbanilides, such as 3-(4-chlorophenyl)-1-(3,4-dichlorphenyl)urea sold under the tradename Triclocarban® by Ciba-Geigy, Florham Park, N.J.; 3-trifluoromethyl-4,4'-dichlorocarbanilide; and 3,3',4-trichlorocarbanilide.

Examples of polymeric antimicrobial agents that can be used in the compositions defined herein include, but are not limited to: polyhexamethylene biguanide hydrochloride; and poly(iminoimidocarbonyl iminoimidocarbonyl iminohexamethylene hydrochloride), which is sold under the tradename Vantocil® IB.

Examples of thazolines that can be used in the compositions defined herein include, but are not limited to that sold under the tradename Micro-Check®; and 2-n-octyl-4-isothiazolin-3-one, which is sold under the tradename Vinyzene® IT-3000 DIDP.

Examples of trichloromethylthioimides that can be used in the compositions as defined herein include, but are not limited to: N-(trichloromethylthio)phthalimide, which is sold under the tradename Fungitrol®; and N-trichloromethylthio-4-cyclohexene-1,2-dicarboximide, which is sold under the tradename Vancide®.

Examples of natural antimicrobial agents that can be used in the compositions as defined herein include, but are not limited to, oils of: anise, lemon, orange, rosemary, wintergreen, thyme, lavender, cloves, hops, tea tree, citronella, wheat, barley, lemongrass, cedar leaf, cedarwood, cinnamon, fleagrass, geranium, sandalwood, violet, cranberry, eucalyptus, vervain, peppermint, gum benzoin, basil, honey, fennel, fir, balsam, menthol, ocmea origanum, hydastis, carradensis, Berberidaceac daceae, Ratanhiae longa, and Curcuma longa. Also included in this class of natural antimicrobial agents are the key chemical components of the plant oils which have been found to provide antimicrobial benefit. These chemicals include, but are not limited to: anethol, catechole, camphene, thymol, eugenol, eucalyptol, ferulic acid, famesol, hinokitiol, tropolone, limonene, menthol, methyl salicylate, carvacol, terpineol, verbenone, berberine, ratanhiae extract, caryophellene oxide, citronellic acid, curcumin, nerolidol, and geraniol.

Examples of metal salts that can be used in the compositions include, but are not limited to, salts of metals in groups 3a-5a, 3b-7b, and 8 of the periodic table. Specific examples of metal salts include, but are not limited to, salts of: aluminum, zirconium, zinc, silver, gold, copper, lanthanum, tin, mercury, bismuth, selenium, strontium, scandium, yttrium, cerium, praseodymiun, neodymium, promethum, samarium, europium, gadolinium, terbium, dysprosium, holmium, erbium, thalium, ytterbium, lutetium, and mixtures thereof. An example of the metal-ion based antimicrobial agent is sold under the tradename HealthShield®, and is manufactured by HealthShield Technology, Wakefield, Mass.

Example of broad-spectrum antimicrobial agents that can be used in the compositions as defined herein include, but are not limited to, those that are recited in other categories of antimicrobial agents herein.

Additional antimicrobial agents that can be used in the methods of the disclosure include, but are not limited to: pyrithiones, and in particular pyrithione-including zinc complexes such as that sold under the tradename Octopirox®; dimethyidimethylol hydantoin, which is sold under the tradename Glydant®; methylchloroisothiazolinone /methylisothiazolinone, which is sold under the tradename Kathon CG®; sodium sulfite; sodium bisulfite; imidazolidinyl urea, which is sold under the tradename Germall 115®; diazolidinyl urea, which is sold under the tradename Germall 11®; benzyl alcohol v2-bromo-2-nitropropane-1,3-diol, which is sold under the tradename Bronopol®; formalin or formaldehyde; iodopropenyl butylcarbamate, which is sold under the tradename Polyphase P101®; chloroacetamide; methanamine; methyldibromonitrile glutaronitrile (1,2-dibromo-2,4-dicyanobutane), which is sold under the tradename Tektamer®; glutaraldehyde; 5-bromo-5-nitro-1,3-dioxane, which is sold under the tradename Bronidox®; phenethyl alcohol; o-phenylphenol/sodium o-phenylphenol sodium hydroxymethylglycinate, which is sold under the tradename Suttocide A®;

polymethoxy bicyclic oxazolidine; which is sold under the tradename Nuosept C®; dimethoxane; thimersal; dichlorobenzyl alcohol; captan; chlorphenenesin; dichlorophene; chlorbutanol; glyceryl laurate; halogenated diphenyl ethers; 2,4,4'-trichloro-2'-hydroxy-diphenyl ether, which is sold under the tradename Triclosan® and is available from Ciba-Geigy, Florham Park, N.J.; and 2,2'-dihydroxy-5,5'-dibromo-diphenyl ether.

Additional antimicrobial agents that can be used in the methods of the disclosure include those disclosed by U.S. Pat. Nos. 3,141,321; 4,402,959; 4,430,381; 4,533,435; 4,625,026; 4,736,467; 4,855,139: 5,069,907; 5,091,102; 5,639,464; 5,853,883; 5,854,147; 5,894,042; and 5,919,554; and U.S. Pat. Appl. Publ. Nos. 2004/0009227 and 2011/0081530.

(e) Collagens and Agents that Promote Collagen Synthesis

According to some embodiments, the biophotonic compositions, methods and uses of the present disclosure may optionally further comprise one or more collagens and/or agents that promote collagen synthesis. Collagen is a fibrous protein produced in dermal fibroblast cells and forming 70% of the dermis and benefits all stages of the wound healing process. Thus, collagens and agents that promote collagen synthesis may also be useful in the present disclosure. Agents that promote collagen synthesis (i.e., pro-collagen synthesis agents) include amino acids, peptides, proteins, lipids, small chemical molecules, natural products and extracts from natural products.

For instance, it was discovered that intake of vitamin C, iron, and collagen can effectively increase the amount of collagen in skin or bone. See, e.g., U.S. Patent Application Publication No. 20090069217.

Examples of the vitamin C include an ascorbic acid derivative such as L-ascorbic acid or sodium L-ascorbate, an ascorbic acid preparation obtained by coating ascorbic acid with an emulsifier or the like, and a mixture containing two or more of those vitamin Cs at an arbitrary rate. In addition, natural products containing vitamin C such as acerola and lemon may also be used. Examples of the iron preparation include: an inorganic iron such as ferrous sulfate, sodium ferrous citrate, or ferric pyrophosphate; an organic iron such as heme iron, ferritin iron, or lactoferrin iron; and a mixture containing two or more of those irons at an arbitrary rate. In addition, natural products containing iron such as spinach or liver may also be used. Moreover, examples of the collagen include: an extract obtained by treating bone, skin, or the like of a mammal such as bovine or swine with an acid or alkaline; a peptide obtained by hydrolyzing the extract with a protease such as pepsin, trypsin, or chymotrypsin; and a mixture containing two or more of those collagens at an arbitrary rate. Collagens extracted from plant sources may also be used.

Additional pro-collagen synthesis agents are described, for example, in U.S. Patent Patents Nos. 7598291, 7722904, 6203805, 5529769, and U.S. Patent Application Publication Nos. 20060247313, 20080108681, 20110130459, 20090325885, and 20110086060.

(f) Healing factors

Healing factors comprise compounds that promote or enhance the healing or regenerative process of the tissues on the application site of the composition. During the photoactivation of the composition, there is an increase of the absorption of molecules at the treatment site. An augmentation in the blood flow at the site of treatment is observed for an extent period of time. An increase in the lymphatic drainage and a possible change in the osmotic equilibrium due to the dynamic interaction of the free radical cascades can be enhanced or even fortified with the inclusion of healing factors. Suitable healing factors for the compositions, methods and uses of the present disclosure include, but are not limited to:

*Hyaluronic acid (Hyaluronan or Hyaluronate)*

Hyaluronic acid (hyaluronan or hyaluronate) is a non-sulfated glycosaminoglycan, distributed widely throughout connective, epithelial and neural tissues. It is one of the primary components of the extracellular matrix, and contributes significantly to cell proliferation and migration. Hyaluronan is a major component of the skin, where it is involved in tissue repair. While it is abundant in extracellular matrices, it contributes to tissue hydrodynamics, movement and proliferation of cells and participates in a wide number of cell surface receptor interactions, notably those including primary receptor CD44. The hyaluronidase enzymes degrade hyaluronan and there are at least seven types of hyaluronidase-like enzymes in humans, several of which are tumor suppressors. The degradation products of hyaluronic acid, the oligosaccharides and the very-low molecular weight hyaluronic acid, exhibit pro-angiogenic properties. In addition, recent studies show that hyaluronan fragments, but not the native high molecular mass of hyaluronan, can induce inflammatory responses in macrophages and dendritic cells in tissue injury. Hyaluronic acid is well suited to biological applications targeting the skin. Due to its high biocompatibility, it is used to stimulate tissue regeneration. Current studies evidenced hyaluronic acid appearing in the early stages of healing to physically create room for white blood cells that mediate the immune response. It is used in the synthesis of biological scaffolds for wound healing applications and in wrinkle treatment. In

certain embodiments, the composition includes hyaluronic acid in the range of less than about 2% by weight of the total composition hyaluronic acid. In some embodiments, hyaluronic acid is present in an amount from about 0.001% to about 2%, or from about 0.002% to about 2%, or from about 0.002% to about 1 % by weight of the total composition.

*Glucosamine*

Glucosamine is one of the most abundant monosaccharides in human tissues and a precursor in the biological synthesis of glycosylated proteins and lipids. It is commonly used in the treatment of osteoarthritis. The common form of glucosamine used is its sulfate salt. Glucosamine shows a number of effects including, anti-inflammatory activity, stimulation of the synthesis of proteoglycans and the synthesis of proteolytic enzymes. A suitable range of concentration over which glucosamine can be used in the present composition is from less than about 5% by weight of the total composition. In some embodiments, glucosamine is present in an amount from about 0.0001 % to about 5%, or from about 0.0001% to about 3%, or from about 0.001% to about 3%, or from about 0.001% to about 1%, or about 0.01% to about 1%, or about 1% to about 3% by weight of the total composition.

*Allantoin*

Allantoin is a diureide of glyosilic acid. It has keratolytic effect, increases the water content of the extracellular matrix, enhances the desquamation of the upper layers of dead (apoptotic) skin cells, and promotes skin proliferation and wound healing. In certain embodiments, the composition includes in the range of less than about 1% by weight of the total composition allantoin. In some embodiments, allantoin is present in an amount from about 0.001% to about 1%, or from about 0.002% to about 1%, or from about 0.02% to about 1%, or from about 0.02% to about 0.5% by weight of the total composition.

Also, saffron can act as both a photon-transfer agent and a healing factor.

(g) Chelating agents

Chelating agents can be included to promote smear layer removal in closed pockets and difficult to reach lesions. Chelating agents act as a metal ion quencher and as a buffer. Suitable chelating agents for the compositions, methods and uses of the disclosure include, but are not limited to:

*Ethylenediaminotetraacetic acid (EDTA)*

Ethylenediaminotetraacetic acid (EDTA) is an amino acid and is used to sequester di- and trivalent metal ions. EDTA binds to metals via four carboxylate and two amine groups.

EDTA forms especially strong complexes with Mn(III), Fe(III), Cu(III), Co(III). It is used to buffer solutions.

*Ethylene glycol tetraacetic acid (EGTA)*

Ethylene glycol tetraacetic acid (EGTA) is related to EDTA, but with a much higher affinity for calcium than magnesium ions. It is useful for making buffer solutions that resemble the environment inside living cells.

(h) Additional Components

The compositions, methods, and uses of the disclosure can also include other ingredients such as humectants (e.g., glycerine, ethylene glycol, and propylene glycol), preservatives such as parabens, and pH adjusters such as sodium hydroxide, sodium bicarbonate, and HCl. In some embodiments, the pH of the composition is in or adjusted to the range of about 4 to about 10. In some embodiments, the pH of the composition is in or adjusted to the range of about 4 to about 9. In some embodiments, the pH of the composition is in or adjusted to the range of about 4 to about 8. In some embodiments, the pH of the composition is within the range of about 4 to about 7. In some embodiments, the pH of the composition is within the range of about 4 to about 6.5. In some embodiments, the pH of the composition is within the range of about 4 to about 6. In some embodiments, the pH of the composition is within the range of about 4 to about 5.5. In some embodiments, the pH of the composition is within the range of about 4 to about 5. In some embodiments, the pH of the composition is within the range of about 5.0 to about 8.0. In some embodiments, the pH of the composition is within the range of about 6.0 to about 8.0. In some embodiments, the pH of the composition is within the range of about 6.5 to about 7.5. In some embodiments, the pH of the composition is within the range of about 5.5 to about 7.5.

In some embodiments, the pH of the composition is in or adjusted to the range of 4 to 10. In some embodiments, the pH of the composition is in or adjusted to the range of 4 to 9. In some embodiments, the pH of the composition is in

or adjusted to the range of 4 to 8. In some embodiments, the pH of the composition is within the range of 4 to 7. In some embodiments, the pH of the composition is within the range of 4 to 6.5. In some embodiments, the pH of the composition is within the range of 4 to 6. In some embodiments, the pH of the composition is within the range of 4 to 5.5. In some embodiments, the pH of the composition is within the range of 4 to 5. In some embodiments, the pH of the composition is within the range of 5.0 to 8.0. In some embodiments, the pH of the composition is within the range of 6.0 to 8.0. In some embodiments, the pH of the composition is within the range of 6.5 to 7.5. In some embodiments, the pH of the composition is within the range of 5.5 to 7.5.

In some embodiments, the compositions of the disclosure also include an aqueous substance (water) or an alcohol. Alcohols include, but are not limited to, ethanol, propanol, isopropanol, butanol, iso-butanol, t-butanol or pentanol. In some embodiments, the chromophore or combination of chromophores is in solution in a medium of the biophotonic composition. In some embodiments, the chromophore or combination of chromophores is in solution in a medium of the biophotonic composition, wherein the medium is an aqueous substance.

*(3) Optical properties of the Biophotonic Compositions*

In certain embodiments, biophotonic compositions of the present disclosure are substantially transparent or translucent. The % transmittance of the biophotonic composition can be measured in the range of wavelengths from 250 nm to 800 nm using, for example, a Perkin-Elmer Lambda 9500 series UV-visible spectrophotometer. In some embodiments, transmittance within the visible range is measured and averaged. In some other embodiments, transmittance of the biophotonic composition is measured with the chromophore(s) omitted. As transmittance is dependent upon thickness, the thickness of each sample can be measured with calipers prior to loading in the spectrophotometer. Transmittance values can be normalized according to:

$$F_{T-corr}(\lambda, t_2) = [e^{-\sigma_t(\lambda)t_1}]^{\frac{t_2}{t_1}} = [F_{T-corr}(\lambda, t_1)]^{\frac{t_2}{t_1}},$$

where $t_1$=actual specimen thickness, $t_2$=thickness to which transmittance measurements can be normalized. In the art, transmittance measurements are usually normalized to 1 cm.

In certain embodiments, the biophotonic compositions are substantially opaque. In these embodiments, the biophotonic compositions may include light transmitting structures such as fibres, particles, networks, which are made of materials which can transmit light. The light transmitting structures can be waveguides such as optical fibres.

In some embodiments, the biophotonic composition has a transmittance that is more than about 20%, about 25%, about 30%, about 35%, about 40%, about 45%, about 50%, about 55%, about 60%, about 65%, about 70%, or about 75% within the visible range. In some embodiments, the transmittance exceeds 40%, 41%, 42%, 43%, 44%, or 45% within the visible range.

*(4) Forms of the Biophotonic Compositions*

The biophotonic compositions of the present disclosure may be a liquid, a gel, a cream, a paste, a putty, a semi-solid, or a solid. Biophotonic compositions in the liquid, gel, cream, paste or putty form can be applied by spreading, spraying, smearing, dabbing or rolling the composition on the target tissue. Biophotonic compositions of the putty, semi-solid or solid forms may be deformable. They may be elastic or non-elastic (i.e., flexible or rigid). The biophotonic compositions, for example, may be in a peel-off form ('peelable') to provide ease and speed of use. In certain embodiments, the tear strength and/or tensile strength of the peel-off form is greater than its adhesion strength. This may help handleability of the composition. It will be recognized by one of skill in the art that the properties of the peel-off biophotonic composition such as cohesiveness, flexibility, elasticity, tensile strength, and tearing strength, can be determined and/or adjusted by methods known in the art such as by selecting suitable thickening agents and adapting their relative ratios.

The biophotonic composition may be in a pre-formed shape. In certain embodiments, the pre-formed shape is in the form of, including, but not limited to, a film, a face mask, a patch, a dressing, or bandage. The biophotonic composition can be configured with a shape and/or size for application to a desired portion of a subject's body. For example, the biophotonic composition can be shaped and sized to correspond with a desired portion of the body to receive the biophotonic treatment. Such a desired portion of the body can be selected from, but not limited to, the group consisting of a skin, head, forehead, scalp, nose, cheeks, lips, ears, face, neck, shoulder, arm pit, arm, elbow, hand, finger, abdomen, chest, stomach, back, buttocks, sacrum, genitals, legs, knee, feet, toes, nails, hair, soft tissues, any boney prominences, and combinations thereof, and the like. The biophotonic composition may also be configured to be applied internally to a subject's body, such as on the luminal surface of a body cavity or organ of a subject, or be configured to be fitted or juxtapositioned to cover a substantial portion of the subject's external body surface or surface

of a limb or other extremity. Thus, the biophotonic composition of the disclosure can be shaped and sized to be applied to any portion of tissue on a subject's body. For example, the biophotonic composition can be provided in the form of sock, hat, glove or mitten. In certain aspects, the biophotonic composition forms part of a composite and can include fibres, particulates, non-biophotonic layers or biophotonic layers with the same or different compositions. The biophotonic compositions of the present disclosure may have a thickness of, or be applied with a thickness of, from about 0.1 mm to about 50 mm, about 0.5 mm to about 20 mm, or about 1 mm to about 10 mm. It will be appreciated that the thickness of the biophotonic compositions will vary based on the intended use. In some embodiments, the biophotonic composition has a thickness of from about 0.1-1 mm. In some embodiments, the biophotonic composition has a thickness of about 0.5-1.5 mm, about 1-2 mm, about 1.5-2.5 mm, about 2-3 mm, about 2.5-3.5 mm, about 3-4 mm, about 3.5-4.5 mm, about 4-5 mm, about 4.5-5.5 mm, about 5-6 mm, about 5.5-6.5 mm, about 6-7 mm, about 6.5-7.5 mm, about 7-8 mm, about 7.5-8.5 mm, about 8-9 mm, about 8.5-9.5 mm, about 9-10 mm, about 10-11 mm, about 11-12 mm, about 12-13 mm, about 13-14 mm, about 14-15 mm, about 15-16 mm, about 16-17 mm, about 17-18 mm, about 18-19 mm, about 19-20 mm, about 20-22 mm, about 22-24 mm, about 24-26 mm, about 26-28 mm, about 28-30 mm, about 30-35 mm, about 35-40 mm, about 40-45 mm, or about 45-50 mm. In some embodiments, the biophotonic composition has a thickness of 0.1 mm to 50 mm, 0.5 mm to 20 mm, or 1 mm to 10 mm. In some embodiments, the biophotonic composition has a thickness of from 0.1-1 mm. In some embodiments, the biophotonic composition has a thickness of 0.5-1.5 mm, 1-2 mm, 1.5-2.5 mm, 2-3 mm, 2.5-3.5 mm, 3-4 mm, 3.5-4.5 mm, 4-5 mm, 4.5-5.5 mm, 5-6 mm, 5.5-6.5 mm, 6-7 mm, 6.5-7.5 mm, 7-8 mm, 7.5-8.5 mm, 8-9 mm, 8.5-9.5 mm, 9-10 mm, 10- 11 mm, 11-12 mm, 12-13 mm, 13-14 mm, 14-15 mm, 15-16 mm, 16-17 mm, 17-18 mm, 18- 19 mm, 19-20 mm, 20-22 mm, 22-24 mm, 24-26 mm, 26-28 mm, 28-30 mm, 30-35 mm, 35- 40 mm, 40-45 mm, or 45-50 mm.

(5) Compositions for Use

The biophotonic compositions of the present disclosure may have cosmetic and/or medical benefits. They can be used to promote skin rejuvenation and skin conditioning, promote the treatment of a skin disorder such as acne, eczema or psoriasis, promote tissue repair and bone repair, and promote wound healing including periodontitis pockets. They can be used to treat acute inflammation. Acute inflammation can present itself as pain, heat, redness, swelling and loss of function, and includes inflammatory responses such as those seen in allergic reactions such as those to insect bites e.g., mosquito, bees, wasps, poison ivy, or post-ablative treatment.

In certain embodiments, the present disclosure provides a biophotonic composition for use in a method for providing skin rejuvenation or for improving skin condition, treating a skin disorder, preventing or treating scarring (e.g., post-surgical scarring) and ameliorating healing, debriding wounds and/or skin, and/or accelerating wound healing and/or tissue repair, the method comprising: applying a biophotonic composition of the present disclosure to the area of the skin or tissue in need of treatment, and illuminating the biophotonic composition with light having a wavelength that overlaps with an absorption spectrum of the chromophore(s) present in the biophotonic composition.

Any source of actinic light can be used. The source of actinic light may be a natural source, such as sunlight, or may be a generated source. Any type of halogen, LED or plasma arc lamp, or laser may be suitable source of generated actinic light. The primary characteristic of suitable sources of actinic light will be that they emit light in a wavelength (or wavelengths) appropriate for activating the one or more photoactivators present in the composition. The appropriate wavelength (or wavelengths) may be in the visible range of wavelengths of light, or may be of a shorter wavelength or of a longer wavelength (e.g. infra red) than visible light. In some embodiments, an argon laser is used. In other embodiments, a potassium-titanyl phosphate (KTP) laser (e.g. a GreenLight™ laser) is used. In yet other embodiments, a LED lamp such as a photocuring device is the source of the actinic light. In some embodiments, the LED lamp may comprise LEDs of more than one wavelength, for example, LEDs that emit at a blue light range and other LEDs that emit at the green light or yellow light range or other ranges of light. In yet other embodiments, the source of the actinic light is a source of light having a wavelength between about 200 to about 800 nm. In other embodiments, the source of the actinic light is a source of visible light having a wavelength between about 400 and about 600 nm. In other embodiments, the source of the actinic light is a source of visible light having a wavelength between about 400 and about 700 nm or about 400 nm to about 750 nm. In yet other embodiments, the source of the actinic light is blue light. In yet other embodiments, the source of the actinic light is red light. In yet other embodiments, the source of the actinic light is green light. Furthermore, the source of actinic light should have a suitable power density. Suitable power density for non- collimated light sources (LED, halogen or plasma lamps) are in the range from about 0.1 mW/cm$^2$ to about 200 mW/cm$^2$, or about 30 mW/cm$^2$ to about 150 mW/cm$^2$. Suitable power density for laser light sources are in the range from about 0.5 mW/cm$^2$ to about 0.8 mW/cm$^2$.

In some embodiments, the light has an energy at the subject's skin surface of between about 0.1 mW/cm$^2$ and about 500 mW/cm$^2$, or 0.1-300 mW/cm$^2$, or 0.1-200 mW/cm$^2$, wherein the energy applied depends at least on the condition being treated, the wavelength of the light, the distance of the skin from the light source and the thickness of the biophotonic composition. In certain embodiments, the light at the subject's skin is between about 1 mW/cm$^2$-40

$mW/cm^2$, or about 20 $mW/cm^2$-60 $mW/cm^2$, or about 40 $mW/cm^2$-80 $mW/cm^2$, or about 60 $mW/cm^2$-100 $mW/cm^2$, or about 80 $mW/cm^2$-120 $mW/cm^2$, or about 100 $mW/cm^2$-140 $mW/cm^2$, or about 30 $mW/cm^2$-180 $mW/cm^2$, or about 120 $mW/cm^2$-160 $mW/cm^2$, or about 140 $mW/cm^2$-180 $mW/cm^2$, or about 160 $mW/cm^2$-200 $mW/cm^2$, or about 110 $mW/cm^2$-240 $mW/cm^2$, or about 110 $mW/cm^2$-150 $mW/cm^2$, or about 190 $mW/cm^2$-240 $mW/cm^2$.

The activation of the chromophore(s) within the biophotonic compositions of the disclosure may take place almost immediately on illumination (femto- or pico seconds). A prolonged exposure period may be beneficial to exploit the synergistic effects of the absorbed, reflected and reemitted light of the biophotonic compositions of the present disclosure and its interaction with the tissue being treated. In some embodiments, the time of exposure to actinic light of the tissue or skin or biophotonic composition is a period between 1 minute and 5 minutes. In other embodiments, the time of exposure to actinic light of the tissue or skin or biophotonic composition is a period between 1 minute and 5 minutes. In some other embodiments, the biophotonic composition is illuminated for a period between 1 minute and 3 minutes. In certain embodiments, light is applied for a period of about 1-30 seconds, about 15-45 seconds, about 30-60 seconds, about 0.75-1.5 minutes, about 1-2 minutes, about 1.5-2.5 minutes, about 2-3 minutes, about 2.5-3.5 minutes, about 3-4 minutes, about 3.5-4.5 minutes, about 4-5 minutes, about 5-10 minutes, about 5-9 minutes, about 5-8 minutes, about 10-15 minutes, about 15-20 minutes, about 20-25 minutes, or about 20-30 minutes. In some embodiments, light is applied for a period of 1 second. In some embodiments, light is applied for a period of 5 seconds. In some embodiments, light is applied for a period of 10 seconds. In some embodiments, light is applied for a period of 20 seconds. In some embodiments, light is applied for a period of 30 seconds. In some embodiments, the biophotonic composition is illuminated for a period less than 30 minutes. In some embodiments, the biophotonic composition is illuminated for a period less than 20 minutes. In some embodiments, the biophotonic composition is illuminated for a period less than 15 minutes. In some embodiments, the biophotonic composition is illuminated for a period less than 10 minutes. In some embodiments, the biophotonic composition is illuminated for a period less than 5 minutes. In some embodiments, the biophotonic composition is illuminated for a period less than 1 minute. In some embodiments, the biophotonic composition is illuminated for a period less than 30 seconds. In some embodiments, the biophotonic composition is illuminated for a period less than 20 seconds. In some embodiments, the biophotonic composition is illuminated for a period less than 10 seconds. In some embodiments, the biophotonic composition is illuminated for a period less than 5 seconds. In some embodiments, the biophotonic composition is illuminated for a period less than 1 second. The treatment time may range up to about 90 minutes, about 80 minutes, about 70 minutes, about 60 minutes, about 50 minutes, about 40 minutes, about 30 minutes or about 20 minutes. It will be appreciated that the treatment time can be adjusted in order to maintain a dosage by adjusting the rate of fluence delivered to a treatment area. For example, the delivered fluence may be about 4 $J/cm^2$ to about 60 $J/cm^2$, about 10 $J/cm^2$ to about 60 $J/cm^2$, about 10 $J/cm^2$ to about 50 $J/cm^2$, about 10 $J/cm^2$ to about 40 $J/cm^2$, about 10 $J/cm^2$ to about 30 $J/cm^2$, about 20 $J/cm^2$ to about 40 $J/cm^2$, about 15 $J/cm^2$ to 25 $J/cm^2$, or about 10 $J/cm^2$ to about 20 $J/cm^2$. The delivery fluence may also be adjusted in terms of levels of singlet oxygen released.

In certain embodiments, the biophotonic compositions of the disclosure may be re- illuminated at certain intervals, such as 1, 2, 3, 4, 5, 6, 7, 8, 9, 10, 11, 12, 24, 36, or 48 hours. In yet other embodiments, the source of actinic light is in continuous motion over the treated area for the appropriate time of exposure. In yet other embodiments, the biophotonic compositions of the disclosure may be illuminated until the biophotonic composition is at least partially photobleached or fully photobleached.

In some embodiments, the chromophore(s) in the biophotonic compositions of the disclosure can be photoexcited by ambient light including from the sun and overhead lighting. In some embodiments, the chromophore(s) can be photoactivated by light in the visible range of the electromagnetic spectrum. The light can be emitted by any light source such as sunlight, light bulb, an LED device, electronic display screens such as on a television, computer, telephone, mobile device, flashlights on mobile devices.

Any source of light can be used. For example, a combination of ambient light and direct sunlight or direct artificial light may be used. Ambient light can include overhead lighting such as LED bulbs, fluorescent bulbs, and indirect sunlight. In the methods and uses of the present disclosure, the biophotonic compositions may be removed from the skin following application of light. In some embodiments, the biophotonic composition is peeled off, or is washed off, the tissue being treated after a treatment time. In other embodiments, the biophotonic composition is left on the tissue for an extended period of time and re-activated with direct or ambient light at appropriate times to treat the condition.

In certain embodiments, the biophotonic compositions can be applied to the tissue, such as on the face or wound, once, twice, three times, four times, five times or six times a week, daily, or at any other frequency. The total treatment time can be one week, two weeks, three weeks, four weeks, five weeks, six weeks, seven weeks, eight weeks, nine weeks, ten weeks, eleven weeks, twelve weeks, or any other length of time deemed appropriate. In certain embodiments, the total tissue area to be treated may be split into separate areas (cheeks, forehead), and each area treated separately. For example, the composition may be applied topically to a first portion, and that portion illuminated with light, and the biophotonic composition then removed. Then the composition is applied to a second portion, illuminated and removed. Finally, the composition is applied to a third portion, illuminated and removed.

In certain embodiments, the biophotonic compositions of the disclosure can be used following wound closure

to optimize scar revision. In this case, the biophotonic compositions may be applied at regular intervals such as once a week, or at an interval deemed appropriate by the physician or any other health care provider.

In certain embodiments, the biophotonic compositions of the disclosure can be used following acne treatment to maintain the condition of the treated skin. In this case, the biophotonic compositions may be applied at regular intervals such as once a week, or at an interval deemed appropriate by the physician or any other health care provider.

In certain embodiments, the biophotonic compositions of the disclosure can be used following ablative skin rejuvenation treatment to maintain the condition of the treated skin. In this case, the biophotonic compositions may be applied at regular intervals such as once a week, or at an interval deemed appropriate by the physician or any other health care provider.

In certain embodiments, the biophotonic compositions of the disclosure can be used to debride a wound or to loosen or remove scaley, dry or dead skin. In this case, the biophotonic compositions may be applied at regular intervals such as once a week, or at an interval deemed appropriate by the physician or any other health care provider.

In certain embodiments, the biophotonic composition of the disclosure can be used to treat bacterial, viral or fungal infections. In this case, the biophotonic compositions may be applied at regular intervals such as once a week, or at an interval deemed appropriate by the physician or any other health care provider.

Additional components may optionally be included in the biophotonic compositions or used in combination with the biophotonic compositions. Such additional components include, but are not limited to, healing factors, antimicrobials, oxygen-rich agents, wrinkle fillers such as botox, hyaluronic acid and polylactic acid, anti-fungal, anti-bacterial, anti-viral agents and/or agents that promote collagen synthesis. These additional components may be applied to the skin in a topical fashion, prior to, at the same time of, and/or after topical application of the biophotonic compositions of the present disclosure. Suitable healing factors comprise compounds that promote or enhance the healing or regenerative process of the tissues on the application site. During the photoactivation of a biophotonic composition of the present disclosure, there may be an increase of the absorption of molecules of such additional components at the treatment site by the skin or the mucosa. In certain embodiments, an augmentation in the blood flow at the site of treatment can observed for a period of time. An increase in the lymphatic drainage and a possible change in the osmotic equilibrium due to the dynamic interaction of the free radical cascades can be enhanced or even fortified with the inclusion of healing factors. Healing factors may also modulate the biophotonic output from the biophotonic composition such as photobleaching time and profile, or modulate leaching of certain ingredients within the composition. Suitable healing factors include, but are not limited to glucosamines, allantoin, saffron, agents that promote collagen synthesis, anti-fungal, anti-bacterial, anti-viral agents and wound healing factors such as growth factors.

(i) Skin Rejuvenation

The biophotonic compositions of the present disclosure may be useful in promoting skin rejuvenation or improving skin condition and appearance. The dermis is the second layer of skin, containing the structural elements of the skin, the connective tissue. There are various types of connective tissue with different functions. Elastin fibers give the skin its elasticity, and collagen gives the skin its strength.

The junction between the dermis and the epidermis is an important structure. The dermal-epidermal junction interlocks forming finger-like epidermal ridges. The cells of the epidermis receive their nutrients from the blood vessels in the dermis. The epidermal ridges increase the surface area of the epidermis that is exposed to these blood vessels and the needed nutrients.

The aging of skin comes with significant physiological changes to the skin. The generation of new skin cells slows down, and the epidermal ridges of the dermal-epidermal junction flatten out. While the number of elastin fibers increases, their structure and coherence decreases. Also the amount of collagen and the thickness of the dermis decrease with the ageing of the skin.

Collagen is a major component of the skin's extracellular matrix, providing a structural framework. During the aging process, the decrease of collagen synthesis and insolubilization of collagen fibers contribute to a thinning of the dermis and loss of the skin's biomechanical properties.

The physiological changes to the skin result in noticeable aging symptoms often referred to as chronological-, intrinsic- and photo-ageing. The skin becomes drier, roughness and scaling increase, the appearance becomes duller, and most obviously fine lines and wrinkles appear. Other symptoms or signs of skin aging include, but are not limited to, thinning and transparent skin, loss of underlying fat (leading to hollowed cheeks and eye sockets as well as noticeable loss of firmness on the hands and neck), bone loss (such that bones shrink away from the skin due to bone loss, which causes sagging skin), dry skin (which might itch), an inability to sweat sufficiently in order to cool the skin, unwanted facial hair, freckles, age spots, spider veins, rough and leathery skin, fine wrinkles that disappear when stretched, loose skin, a blotchy complexion.

The dermal-epidermal junction is a basement membrane that separates the keratinocytes in the epidermis from the extracellular matrix, which lies below in the dermis. This membrane consists of two layers: the basal lamina in contact

with the keratinocytes, and the underlying reticular lamina in contact with the extracellular matrix. The basal lamina is rich in collagen type IV and laminin, molecules that play a role in providing a structural network and bioadhesive properties for cell attachment.

Laminin is a glycoprotein that only exists in basement membranes. It is composed of three polypeptide chains (alpha, beta and gamma) arranged in the shape of an asymmetric cross and held together by disulfide bonds. The three chains exist as different subtypes which result in twelve different isoforms for laminin, including Laminin-1 and Laminin-5.

The dermis is anchored to hemidesmosomes, specific junction points located on the keratinocytes, which consist of $\alpha$-integrins and other proteins, at the basal membrane keratinocytes by type VII collagen fibrils. Laminins, and particularly Laminin-5, constitute the real anchor point between hemidesmosomal transmembrane proteins in basal keratinocytes and type VII collagen.

Laminin-5 synthesis and type VII collagen expression have been proven to decrease in aged skin. This causes a loss of contact between dermis and epidermis, and results in the skin losing elasticity and becoming saggy.

Recently another type of wrinkles, generally referred to as expression wrinkles, got general recognition. These wrinkles require loss of resilience, particularly in the dermis, because of which the skin is no longer able to resume its original state when facial muscles which produce facial expressions exert stress on the skin, resulting in expression wrinkles.

The biophotonic compositions of the present disclosure and methods of the present disclosure promote skin rejuvenation. In certain embodiments, the biophotonic compositions, uses, and methods of the present disclosure promote skin condition such as skin luminosity, reduction of pore size, reducing blotchiness, making even skin tone, reducing dryness, and tightening of the skin. In certain embodiments, the biophotonic compositions, uses, and methods of the present disclosure promote collagen synthesis. In other embodiments, the biophotonic compositions, uses, and methods of the present disclosure may reduce, diminish, retard or even reverse one or more signs of skin aging including, but not limited to, appearance of fine lines or wrinkles, thin and transparent skin, loss of underlying fat (leading to hollowed cheeks and eye sockets as well as noticeable loss of firmness on the hands and neck), bone loss (such that bones shrink away from the skin due to bone loss, which causes sagging skin), dry skin (which might itch), inability to sweat sufficiently to cool the skin, unwanted facial hair, freckles, age spots, spider veins, rough and leathery skin, fine wrinkles that disappear when stretched, loose skin, or a blotchy complexion. In certain embodiments, the biophotonic compositiona, uses, and methods of the present disclosure may induce a reduction in pore size, enhance sculpturing of skin subsections, and/or enhance skin translucence.

In certain embodiments, the biophotonic compositions of the disclosure may be used in conjunction with collagen promoting agents. Agents that promote collagen synthesis (i.e., pro-collagen synthesis agents) include amino acids, peptides, proteins, lipids, small chemical molecules, natural products and extracts from natural products.

For instance, it was discovered that intake of vitamin C, iron, and collagen can effectively increase the amount of collagen in skin or bone. See, e.g., U.S. Patent Application Publication No. 2009/0069217.

Examples of the vitamin C include an ascorbic acid derivative such as L-ascorbic acid or sodium L-ascorbate, an ascorbic acid preparation obtained by coating ascorbic acid with an emulsifier or the like, and a mixture containing two or more vitamin Cs at an arbitrary rate. In addition, natural products containing vitamin C such as acerola and lemon may also be used. Examples of the iron preparation include: an inorganic iron such as ferrous sulfate, sodium ferrous citrate, or ferric pyrophosphate; an organic iron such as heme iron, ferritin iron, or lactoferrin iron; and a mixture containing two or more of those irons at an arbitrary rate. In addition, natural products containing iron such as spinach or liver may also be used. Moreover, examples of the collagen include: an extract obtained by treating bone, skin, or the like of a mammal such as bovine or swine with an acid or alkaline; a peptide obtained by hydrolyzing the extract with a protease such as pepsin, trypsin, or chymotrypsin; and a mixture containing two or more of those collagens at an arbitrary rate. Collagens extracted from plant sources may also be used.

Additional pro-collagen synthesis agents are described, for example, in U.S. Patent Patent Nos 7,598,291; 7,722,904; 6,203,805; 5,529,769; and U.S. Patent Application Publication Nos 2006/0247313; 2008/0108681; 2011/0130459; 2009/0325885: 2011/008600.

(ii) Skin disorders

The biophotonic compositions, uses, and methods of the present disclosure may be used to treat skin disorders that include, but are not limited to, erythema, telangiectasia, actinic telangiectasia, basal cell carcinoma, contact dermatitis, dermatofibrosarcoma protuberans, genital warts, melanoma, merkel cell carcinoma, nummular dermatitis, molloscum contagiosum, psoriasis, psoriatic arthritis, rosacea, scabies, scalp psoriasis, sebaceous carcinoma, squamous cell carcinoma, seborrheic dermatitis, seborrheic keratosis, shingles, tinea versicolor, warts, skin cancer, sunburn, dermatitis, eczema, rashes, impetigo, lichen simplex chronicus, rhinophyma, perioral dermatitis, pseudofolliculitis barbae, drug eruptions, erythema multiforme, erythema nodosum, granuloma annulare, actinic keratosis, purpura, alopecia areata, aphthous stomatitis, dry skin, chapping, xerosis, fungal infections, herpes simplex, intertrigo, keloids, keratoses,

milia, moluscum contagiosum, pityriasis rosea, pruritus, urticaria, and vascular tumors and malformations. Dermatitis includes contact dermatitis, atopic dermatitis, seborrheic dermatitis, nummular dermatitis, generalized exfoliative dermatitis, and statis dermatitis. Skin cancers include melanoma, basal cell carcinoma, and squamous cell carcinoma.

(iii) Acne and Acne Scars

The biophotonic compositions, uses, and methods of the present disclosure may be used to treat acne. As used herein, "acne" means a disorder of the skin caused by inflammation of skin glands or hair follicles. The biophotonic compositions, uses, and methods of the disclosure can be used to treat acne at early pre-emergent stages or later stages where lesions from acne are visible. Mild, moderate and severe acne can be treated with embodiments of the biophotonic compositions and methods. Early pre-emergent stages of acne usually begin with an excessive secretion of sebum or dermal oil from the sebaceous glands located in the pilosebaceous apparatus. Sebum reaches the skin surface through the duct of the hair follicle. The presence of excessive amounts of sebum in the duct and on the skin tends to obstruct or stagnate the normal flow of sebum from the follicular duct, thus producing a thickening and solidification of the sebum to create a solid plug known as a comedone. In the normal sequence of developing acne, hyperkeratinazation of the follicular opening is stimulated, thus completing blocking of the duct. The usual results are papules, pustules, or cysts, often contaminated with bacteria, which cause secondary infections. Acne is characterized particularly by the presence of comedones, inflammatory papules, or cysts. The appearance of acne may range from slight skin irritation to pitting and even the development of disfiguring scars. Accordingly, the biophotonic compositions, uses, and methods of the present disclosure can be used to treat one or more of skin irritation, pitting, development of scars, comedones, inflammatory papules, cysts, hyperkeratinazation, and thickening and hardening of sebum associated with acne.

The biophotonic compositions and methods of the present disclosure may be used to treat various types of acne. Some types of acne include, for example, acne vulgaris, cystic acne, acne atrophica, bromide acne, chlorine acne, acne conglobata, acne cosmetica, acne detergicans, epidemic acne, acne estivalis, acne fulminans, halogen acne, acne indurata, iodide acne, acne keloid, acne mechanica, acne papulosa, pomade acne, premenstral acne, acne pustulosa, acne scorbutica, acne scrofulosorum, acne urticata, acne varioliformis, acne venenata, propionic acne, acne excoriee, gram negative acne, steroid acne, and nodulocystic acne.

Some skin disorders present various symptoms including redness, flushing, burning, scaling, pimples, papules, pustules, comedones, macules, nodules, vesicles, blisters, telangiectasia, spider veins, sores, surface irritations or pain, itching, inflammation, red, purple, or blue patches or discolorations, moles, and/or tumors.

In certain embodiments, the biophotonic compositions of the present disclosure are used in conjunction with systemic or topical antibiotic treatment. For example, antibiotics used to treat acne include tetracycline, erythromycin, minocycline, doxycycline, which may also be used with the compositions and methods of the present disclosure. The use of the biophotonic compositions can reduce the time needed for the antibiotic treatment or reduce the dosage.

(iv) Tissue Repair, Wound Healing

The biophotonic compositions, uses, and methods of the present disclosure may be used to treat wounds, promote wound healing, promote tissue repair, ameliorate post-surgical healing and scarring, and/or prevent or reduce cosmesis including improvement of motor function (e.g. movement of joints). Wounds that may be treated by the biophotonic compositions, uses, and methods of the present disclosure include, for example, injuries to the skin and subcutaneous tissue initiated in different ways (e.g., pressure ulcers from extended bed rest, wounds induced by trauma or surgery, burns, ulcers linked to diabetes or venous insufficiency, wounds induced by conditions such as periodontitis) and with varying characteristics. In certain embodiments, the present disclosure provides biophotonic compositions, uses, and methods for treating and/or promoting the healing of, for example, burns, incisions, excisions, lesions, lacerations, abrasions, puncture or penetrating wounds, surgical wounds, contusions, hematomas, crushing injuries, amputations, sores and ulcers.

Biophotonic compositions, uses, and methods of the present disclosure may be used to treat and/or promote the healing of a fistula. A fistula is an abnormal connection between an organ, vessel, or intestine and another structure, and while a fistula is usually caused by injury or surgery, it may also result from an infection or inflammation, and examples of fistulas that may be treated with the biophotonic composition of the present disclosure include, but are not limited to, a preauricular sinus or cyst, anal fistulas, rectal fistulas, fistulas of the joints, fistulas of the urogenital tract or in relation to the reproductive organs, and fistulas that may occur at any other location on the body.

Biophotonic compositions and methods of the present disclosure may be used to treat and/or promote the healing of chronic cutaneous ulcers or wounds, which are wounds that have failed to proceed through an orderly and timely series of events to produce a durable structural, functional, and cosmetic closure. The vast majority of chronic wounds can be classified into three categories based on their etiology: pressure ulcers, neuropathic (diabetic foot) ulcers and vascular (venous or arterial) ulcers.

For example, the present disclosure provides biophotonic compositions, uses, and methods for treating and/or promoting healing of a diabetic ulcer Diabetic patients are prone to foot and other ulcerations due to both neurologic and vascular complications. Peripheral neuropathy can cause altered or complete loss of sensation in the foot and/or leg. Diabetic patients with advanced neuropathy lose all ability for sharp-dull discrimination. Any cuts or trauma to the foot may go completely unnoticed for days or weeks in a patient with neuropathy. A patient with advanced neuropathy loses the ability to sense a sustained pressure insult, as a result, tissue ischemia and necrosis may occur leading to for example, plantar ulcerations. Microvascular disease is one of the significant complications for diabetics which may also lead to ulcerations. In certain embodiments, biophotonic compositions, uses, and methods of treating a chronic wound are provided here in, where the chronic wound is characterized by diabetic foot ulcers and/or ulcerations due to neurologic and/or vascular complications of diabetes.

In other examples, the present disclosure provides biophotonic compositions, uses, and methods for treating and/or promoting healing of a pressure ulcer. Pressure ulcers include bed sores, decubitus ulcers and ischial tuberosity ulcers and can cause considerable pain and discomfort to a patient. A pressure ulcer can occur as a result of a prolonged pressure applied to the skin. Thus, pressure can be exerted on the skin of a patient due to the weight or mass of an individual. A pressure ulcer can develop when blood supply to an area of the skin is obstructed or cut off for more than two or three hours. The affected skin area can turn red, become painful and necrotic. If untreated, the skin can break open and become infected. A pressure ulcer is therefore a skin ulcer that occurs in an area of the skin that is under pressure from e.g. lying in bed, sitting in a wheelchair, and/or wearing a cast for a prolonged period of time. Pressure ulcers can occur when a person is bedridden, unconscious, unable to sense pain, or immobile. Pressure ulcers often occur in boney prominences of the body such as the buttocks area (on the sacrum or iliac crest), or on the heels of foot.

Additional types of wounds that can be treated by the biophotonic compositions, uses, and methods of the present disclosure include those disclosed by U.S. Pat. Appl. Publ. No. 2009/0220450, which is incorporated herein by reference.

There are three distinct phases in the wound healing process. First, in the inflammatory phase, which typically occurs from the moment a wound occurs until the first two to five days, platelets aggregate to deposit granules, promoting the deposit of fibrin and stimulating the release of growth factors. Leukocytes migrate to the wound site and begin to digest and transport debris away from the wound. During this inflammatory phase, monocytes are also converted to macrophages, which release growth factors for stimulating angiogenesis and the production of fibroblasts.

Second, in the proliferative phase, which typically occurs from two days to three weeks, granulation tissue forms, and epithelialization and contraction begin. Fibroblasts, which are key cell types in this phase, proliferate and synthesize collagen to fill the wound and provide a strong matrix on which epithelial cells grow. As fibroblasts produce collagen, vascularization extends from nearby vessels, resulting in granulation tissue. Granulation tissue typically grows from the base of the wound. Epithelialization involves the migration of epithelial cells from the wound surfaces to seal the wound. Epithelial cells are driven by the need to contact cells of like type and are guided by a network of fibrin strands that function as a grid over which these cells migrate. Contractile cells called myofibroblasts appear in wounds, and aid in wound closure. These cells exhibit collagen synthesis and contractility, and are common in granulating wounds.

Third, in the remodeling phase, the final phase of wound healing which can take place from three weeks up to several years, collagen in the scar undergoes repeated degradation and re-synthesis. During this phase, the tensile strength of the newly formed skin increases.

However, as the rate of wound healing increases, there is often an associated increase in scar formation. Scarring is a consequence of the healing process in most adult animal and human tissues. Scar tissue is not identical to the tissue which it replaces, as it is usually of inferior functional quality. The types of scars include, but are not limited to, atrophic, hypertrophic and keloidal scars, as well as scar contractures. Atrophic scars are flat and depressed below the surrounding skin as a valley or hole. Hypertrophic scars are elevated scars that remain within the boundaries of the original lesion, and often contain excessive collagen arranged in an abnormal pattern. Keloidal scars are elevated scars that spread beyond the margins of the original wound and invade the surrounding normal skin in a way that is site specific, and often contain whorls of collagen arranged in an abnormal fashion.

In contrast, normal skin consists of collagen fibers arranged in a basket-weave pattern, which contributes to both the strength and elasticity of the dermis. Thus, to achieve a smoother wound healing process, an approach is needed that not only stimulates collagen production, but also does so in a way that reduces scar formation.

The biophotonic compositions, uses, and methods of the present disclosure may promote wound healing by debriding the wound, disinfecting the wound, disrupting any biofilm present on the wound, promoting the formation of substantially uniform epithelialization; promoting collagen synthesis; promoting controlled contraction; and/or by reducing the formation of scar tissue. In certain embodiments, the biophotonic compositions, uses, and methods of the present disclosure may promote wound healing by promoting the formation of substantially uniform epithelialization. In some embodiments, the biophotonic compositions, uses, and methods of the present disclosure promote collagen synthesis. In some other embodiments, the biophotonic compositions, uses, and methods of the present disclosure promote controlled contraction. In certain embodiments, the biophotonic compositions, uses, and methods of the present disclosure

promote wound healing, for example, by reducing the formation of scar tissue.

The biophotonic compositions of the present disclosure may also be used in combination with negative pressure assisted would closure devices and systems.

In certain embodiments, the biophotonic composition is kept in place for up to one, two or three weeks, and illuminated with light which may include ambient light at various intervals. In this case, the composition may be covered up in between exposure to light with an opaque material or left exposed to light. In certain embodiments, the biophotonic composition is removed after each treatment.

*(v) Oral Diseases*

The biophotonic compositions, uses, and methods of the present disclosure may be used to treat various oral diseases. Such oral diseases include but are not limited to:

Gingivitis

The biophotonic compositions, uses, and methods of the present disclosure may be used to treat gingivitis. Gingivitis is a disorder that is defined by the inflammation of the gums, and is characterized as a periodontal disease, which is characterized by the destruction of the gums, tissue, tooth sockets, and ligaments which create the structure that holds the teeth in place. Gingivitis is one of the first stages of serious periodontal disease.

The symptoms of gingivitis include swollen gums, mouth sores, a bright red or purple appearance to the gums, shiny gums, gums that are painless except when touched, and bleeding gums. Often the first signs of gingivitis have no symptoms except for visual symptoms and it is likely only to be diagnosed by a dental professional.

Periodontal disease

The biophotonic compositions, uses, and methods of the present disclosure may be used to treat periodontal disease. Periodontal disease is also known as trench mouth. Periodontal disease leads to severe gingivitis and can cause gums to bleed, ooze pus, is highly painful, and often leads to premature tooth loss. While most developed nations have fewer cases of periodontal disease, it does still exist simply due to the high number of employed, working class Americans who are not given dental insurance as part of their benefits package. Dental work is very expensive, and not all patients can afford good dental care.

Periodontal disease is more prevalent in developing nations and in most cases, a professional cleaning and antibiotics can clear up most cases of periodontal disease. However, if left untreated the infection can spread throughout the body and can lead to serious health complications.

Symptoms of periodontal disease include painful gums, bad breath, a foul taste to the mouth, fever, gums that bleed with only mild amounts of pressure, crater sized canker sores between the teeth and gums, swollen lymph nodes around the head, neck, or jaw, a gray film on the gums, red gums, swollen gums, and pain when eating and swallowing.

Periodontitis

The biophotonic compositions, uses, and methods of the present disclosure may be used to treat periodontitis. Periodontitis or *Pyorrhea alveolaris* is the inflammation of the periodontium which comprises tissues supporting the teeth in the oral cavity. Parts included in the periodontium are the gingiva (gum tissue), the alveolar bone which are sockets where teeth are attached, the cementum or outer layer of teeth roots and the periodontal ligaments or PDL composed of connective tissue fibers linking the gingival and cementum to the alveolar bone. The condition is described as the progressive loss of bone around teeth leading to loose teeth or loss of teeth if left unattended. There are different causes for the disease in which bacteria is the most common. Periodontitis is considered as an advanced phase of gum disease since it already involves bone loss in the area. It is the effect of mild gingivitis being left untreated. Due to the presence of bacterial infection, the body can also respond negatively to it leading to further complications. The condition is one of the leading causes of tooth loss among adults, affecting around 50% of everyone over the age of 30.

Signs and symptoms arise due to the unstable anchoring of teeth as well as the presence of microorganisms. Gums occasionally or frequently bleed or turn red while brushing teeth, using dental floss, biting into food, chewing or touching with fingers. Gums swell or develop pus occasionally as well. The affected individual likely has halitosis or bad breath and have a lingering metallic or tinny taste inside the mouth. Teeth seem longer and sharper due to gingival recession which partly may also be caused by hard brushing. If enzymes called collagenases have begun destroying collagen, the person will have deep pockets between the teeth and gums.

During the early stages of periodontal disease, only a few signs and symptoms may be noticeable. Aggressive periodontitis may affect younger individuals and can occur in episodes. Some episodes may present very mild symptoms while others may be very severe. The signs and symptoms especially in the case of chronic periodontitis are usually progressive in nature.

## Oral thrush

The biophotonic compositions, uses, and methods of the present disclosure may be used to treat oral thrush. Oral thrush is the condition where the fungus *Candida albicans* grows rapidly and uncontrollably in the mouth. The bacterium known as flora keeps the growth of *Candida albicans* under control in a healthy body. Oral thrush presents with creamy white paste that covers the tongue, and can spread rapidly to the roof of the mouth, gums, back of the throat, tonsils, and the inside of the cheeks. Babies, toddlers, older adults, and patients whose immune systems have been somehow compromised are most likely to come down with oral thrush.

Symptoms of oral thrush begin with a white pasty covering over the tongue and inside of the cheeks. As the oral thrush continues to develop, it can cause a mild amount of bleeding if the tongue is scraped or when the patient brushes their teeth. These symptoms may develop very quickly, but thrush can last for months. If the lesions of oral thrush spread down the esophagus, the patient may develop addition symptoms such as difficulty swallowing, the sensation of food being caught in the throat or the middle of the chest, and a fever should the infection continue to spread past the esophagus.

## Lichen planus

The biophotonic compositions, uses, and methods of the present disclosure may be used to treat lichen planus. Lichen planus is most often defined as an oral disease that affects the lining of the mouth with inflammation. Lichen planus is most often recognized as a rash that irritates the tissue of the oral cavity. Most patients come down with their first case between the age of 45 and 60, although a slowly increasing number of reports dealing with younger patients have trickled in. While lichen planus is most often associated with the interior of the cheeks, many cases will find the entire mouth is affected, including the gums, the tongue, the lips, and in rare cases, the throat or esophagus. Lichen planus also occurs on the skin, as a skin disease, and often must be referred to specifically as skin lichen planus to differentiate between the oral type.

Lichen planus is a self-contained disease that can last for weeks, months, and in some cases, years. It is not contagious. It is often mistaken for genital diseases, as the genitalia are often the most noticeably affected during the early development stage. Because the symptoms and outbreaks occur rapidly and then disappear, often for weeks, treatment is difficult. While some patients find great relief in cool compresses or tub soaks and cool baths, most patients require medical treatment in order to relieve their symptoms.

## Stomatitis

The biophotonic compositions, uses, and methods of the present disclosure may be used to treat stomatitis. Stomatitis basically means inflammation of the mouth, but more specifically, stomatitis is the inflammation of the mucous lining of the mouth which may include the gums, tongue, cheeks, lips and the floor or roof of the mouth. There are different types of stomatitis and classification is based on how the disease was acquired by a person. The two types of stomatitis are contact stomatitis and aphthous stomatitis. Contact stomatitis is an inflammation of the oral mucosa caused by coming in contact with allergens or irritants. It is classified by its pattern of distribution, etiologic factors, and clinical features. There some cases of contact stomatitis that are left undetected because of the lack of clinical signs. Anybody can have contact stomatitis regardless of race, age and sex. Although it has been observed that it is more common in the elders.

Aphthous stomatitis, also known as canker sore or aphthous ulcers, has an unknown etiology. Just like contact stomatitis, canker sore affects the oral mucosa. An aphthous ulcer is a type of oral ulcer, which presents as a painful open sore inside the mouth or upper throat (including the uvula) caused by a break in the mucous membrane. The condition is also known as Sutton’s Disease, especially in the case of major, multiple, or recurring ulcers. The ulcers can be described as shallow, discrete, and painful and are usually visible on the mucous membranes that are unattached. This type of stomatitis, just like contact stomatitis, is self-limited and do not usually cause complications. The normal size of ulcers may last for 1 to 2 weeks but larger ulcers may last for months.

## Herpes simplex lesions

The biophotonic compositions, uses, and methods of the present disclosure may be used to treat herpes simplex lesions. Herpes simplex is a viral disease caused by herpes simplex viruses; both herpes simplex virus 1 (HSV-1) and herpes simplex virus 2 (HSV-2) cause herpes simplex. Infection with the herpes virus is categorized into one of several distinct disorders based on the site of infection. Oral Herpes, the visible symptoms of which are colloquially called cold sores, and infects the face and mouth. Oral herpes is the most common form of herpes simplex virus infection.

Other oral inflammatory lesions

The compositions and methods of the present disclosure may be used to treat other types of oral inflammation, including but not limited to oral mucositis, oral ulcers caused by viral, bacterial, fungal or protozoan infections, or caused by disorders of the immune system (immunodeficiency, autoimmunity, or allergy). Also included is oral submucous fibrosis, a chronic debilitating disease of the oral cavity characterized by inflammation and progressive fibrosis of the submucosal tissues. Also included is glossitis, an inflammation or infection of the tongue. It causes the tongue to swell and change color.

*(vi) Bone Regeneration*

The biophotonic compositions, uses, and methods of the present disclosure are useful for bone reconstruction and/or regeneration. Without being bound by theory, the compositions of the disclosure may help promote the growth, recruitment and survival of bone tissue at a particular site. In use, the composition may be implanted at a site at which bone growth is desired, e.g. to treat a disease, defect or location of trauma, and/or to promote artificial arthrodesis. Bone repair sites that can be treated with the composition of the disclosure include, but are not limited to, those resulting from injury, defects brought about during the course of surgery, infection, malignancy or developmental malformation. The compositions can be used in a wide variety of orthopedic, periodontal, neurosurgical and oral and maxillofacial surgical procedures including, but not limited to: the repair of simple and compound fractures and non-unions; external and internal fixations; joint reconstructions such as arthrodesis; general arthroplasty; cup arthroplasty of the hip; femoral and humeral head replacement; femoral head surface replacement and total joint replacement; repairs of the vertebral column including spinal fusion and internal fixation; tumor surgery, e.g., deficit filing; discectomy; laminectomy; excision of spinal cord tumors; anterior cervical and thoracic operations; repairs of spinal injuries; scoliosis, lordosis and kyphosis treatments; intermaxillary fixation of fractures; mentoplasty; temporomandibular joint replacement; alveolar ridge augmentation and reconstruction; inlay osteoimplants; implant placement and revision; sinus lifts; cosmetic enhancement; etc. For any of these potential applications, compositions of the disclosure may be applied directly to a site where bone reconstruction is needed. Accessing this site may, in some cases, require surgical intervention to expose the site. However, in some cases, the site is already exposed or can be accessed without the need for surgical intervention.

Any bone disease or disorder may be treated using the composition of the present disclosure including genetic diseases, congenital abnormalities, fractures, iatrogenic defects, bone cancer, bone metastases, inflammatory diseases (e.g. rheumatoid arthritis), autoimmune diseases, metabolic diseases, and degenerative bone disease (e.g., osteoarthritis). In certain embodiments, the compositions are formulated for the repair of a simple fracture, compound fracture, or non-union; as an external fixation device or internal fixation device; for joint reconstruction, arthrodesis, arthroplasty, or cup arthroplasty of the hip; for femoral or humeral head replacement; for femoral head surface replacement or total joint replacement; for repair of the vertebral column, spinal fusion or internal vertebral fixation; for tumor surgery; for deficit filling; for discectomy; for laminectomy; for excision of spinal tumors; for an anterior cervical or thoracic operation; for the repairs of a spinal injury; for scoliosis, for lordosis or kyphosis treatment; for intermaxillary fixation of a fracture; for mentoplasty; for temporomandibular joint replacement; for alveolar ridge augmentation and reconstruction; as an inlay osteoimplant; for implant placement and revision; for sinus lift; for a cosmetic procedure; for revision surgery; for revision surgery of a total joint arthroplasty; and for the repair or replacement of the ethmoid, frontal, nasal, occipital, parietal, temporal, mandible, maxilla, zygomatic, cervical vertebra, thoracic vertebra, lumbar vertebra, sacrum, rib, sternum, clavicle, scapula, humerus, radius, ulna, carpal bones, metacarpal bones, phalanges, ilium, ischium, pubis, femur, tibia, fibula, patella, calcaneus, tarsal bones, or metatarsal bones. The composition may be made flowable before it is administered to a subject. This allows the composition to fit into irregularly shaped sites. In certain embodiments, the composition is injected or extruded into a tissue site (e.g., a bony defect or bone cavity). For example, the composition may be injected using a needle and syringe. The syringe may be driven by hand or mechanically. In some embodiments, the mixture is injected percutaneously. A bony injection site may be some distance from the skin, necessitating a longer needle. In other embodiments, the injection site may be exposed, for example, during surgery. In these cases a very short cannula may suffice for delivery of the mixture, and a wider bore cannula may be appropriate.

*(6) Kits*

The present disclosure also provides kits a containing the biophotonic compositions and/or providing any of the components required for preparing biophotonic compositions of the present disclosure.

In some embodiments, the kit includes a biophotonic composition of the present disclosure. In some embodiments, the kit includes containers comprising the components that can be used to make the biophotonic composition of the present disclosure. The different components making up the biophotonic compositions of the present disclosure may be provided in separate containers. For example, in embodiments where the biophotonic composition comprises a

peroxide source, the peroxide or peroxide precursor of the biophotonic composition may be provided in a container separate from the chromophore(s). Examples of such containers are dual chamber syringes, dual chamber containers with removable partitions, sachets with pouches, and multiple-compartment blister packs. Another example is one of the components being provided in a syringe which can be injected into a container of another component.

In other embodiments, the kit comprises a systemic drug for augmenting the treatment of the biophotonic composition of the present disclosure. For example, the kit may include a systemic or topical antibiotic, hormone treatment (e.g., for acne treatment or wound healing), or a negative pressure device.

The kit may also include instructions for use. The carrier medium may be included together with any of the other three components. In some embodiments, the kit comprises a means for applying the components of the biophotonic compositions such as a spatula, a syringe, or the like.

In certain aspects, there is provided a container comprising a chamber for holding a biophotonic composition, and an outlet in communication with the chamber for discharging the biophotonic composition from the container, wherein the biophotonic composition comprises at least one chromophore in a carrier medium which can form a biophotonic composition after being discharged from the sealed chamber, for example on contact with skin or on illumination with a light. In certain embodiments, the chamber is partitioned such that the chromophore(s), and the peroxide or peroxide precursor are kept in separate compartments until discharged from the container or during discharging from the container.

In certain embodiments, the kit comprises a dressing or a mask. The dressing or mask may be a porous or semi-porous structure for receiving the biophotonic composition. The dressing or mask may also comprise woven or non-woven fibrous materials. The biophotonic composition or its precursor can be incorporated, such as by injection, into the dressing.

In certain embodiments of the kit, the kit may further comprise a light source such as a portable light with a wavelength appropriate to activate the chromophore(s) in the biophotonic composition. The portable light may be battery operated or re-chargeable. The light source may comprise LEDs.

Written instructions on how to use the biophotonic compositions in accordance with the present disclosure may be included in the kit, or may be included on or associated with the containers comprising the compositions or components making up the biophotonic compositions of the present disclosure. The instructions can include information on how to form the biophotonic composition from the individual components or biophotonic composition precursors provided with the kit.

Identification of equivalent biophotonic compositions, methods and kits are well within the skill of the ordinary practitioner and would require no more than routine experimentation, in light of the teachings of the present disclosure.

Variations and modifications will occur to those of skill in the art after reviewing this disclosure. The disclosed features may be implemented, in any combination and subcombinations (including multiple dependent combinations and subcombinations), with one or more other features described herein. The various features described or illustrated above, including any components thereof, may be combined or integrated in other systems. Moreover, certain features may be omitted or not implemented. Examples of changes, substitutions, and alterations are ascertainable by one skilled in the art and could be made without departing from the scope of the information disclosed herein.

## EXAMPLES

The examples below are given so as to illustrate the practice of various embodiments of the present disclosure. They are not intended to limit or define the entire scope of this disclosure.

<u>Example 1: Characterization of Photosynthetic Organism-Derived Chromophores</u>

The solubility, absorbance peaks and emission peaks were determined for Aloe-emodin, Apigenin, Berberine, Caffeic acid, Caffeine, Curcumin, Garcinia acid, Gingerol, Hyperforin, Hypericin, Ellagic Acid, Lycopene, Oleuropein, Piperine, Resveratrol, Sanguinarine, Tannic acid, Theobromine, and Zeaxanthin. The compounds were dissolved in the solvents and at the concentrations indicated in Table 2 and the absorbance and emission peaks were measured (measured using a Flex Station II, Model 384 by Molecular Devices Comp.). These data are shown in Table 2 and indicate that many of these photosynthetic organism-derived chromophores absorb and/or emit light within the range of about 400 nm to about 750 nm.

Table 2

| Compound | Solubilization | Absorbance peak (nm) | Emission peak (nm)** |
|---|---|---|---|
| Aloe-emodin | 10 mg/mL in DMSO | 300 nm and 410 nm (gel); 300 nm-500 nm (liquid) | ex. 460 nm, em. 588 nm (liquid); ex. 300 nm, em. 416 nm and 585 nm; ex. 410 nm, em. 595 nm and 820 nm (liquid); ex. 460 nm, em. 600 nm (gel): ex. 410 nm, em. 610 nm and 822 nm (gel); ex. 300 nm, em. 420 nm and 610 nm (gel) |
| Apigenin | 1 mg/mL in DMSO | 332 nm (sel) | ex. 332 nm, em. 415 nm and 720 nm (liquid); ex. 332 nm em. 603 nm (gel) |
| Berberine chloride hydrate | EtOH/20% DMSO | 346 nm and 428 nm | ex. 460 nm, em. 540 nm (gel + liquid): ex. 340 nm, em. 538 nm (gel): ex. 430 nm, em. 545 nm (liquid) |
| Caffeic acid | EtOH | 300 nm-370 nm (gel): 875 nm (liquid) | ex. 360 nm, em. 452 nm and 728 nm |
| Caffeic acid phenethyl ester (CAPE) | 10 mg/mL in EtOH | -- | ex. 300 nm, em. 430 nm and 807 nm (liquid); ex. 300 nm. em. 415 nm and 765nm (gel) |
| Caffeine | Water | 870 nm | ex. 360 nm. em, 725 nm (gel) |
| Curcumin | 5 mg/mL in EtOH | 440 nm | ex. 460 nm, em. 555 nm (gel); ex. 460 nm, em. 544 nm (liquid); ex. 390 nm, em. 540 nm (liquid): ex. 440 nm, em. 552 nm (gel) |
| Ellagic acid | 1 M NaOH/ 25% DMSO | 345 nm and 432 nm (liquid): 370 nm (gel) | ex. 370 nm, em. 736 nm (gel) |
| Garcinia acid | 10 mg/mL in DMSO | 300 nm (gel and liquid) | ex. 300 nm, em. 378 nm and 747 nm (liquid); ex. 300 nm, em. 378 nm and 743 nm (gel) |
| 10-Gingerol | 10 mg/mL in MeOH | 300 nm (gel and liquid) | ex. 300 nm, em. 380 nm and 753 nm (liquid); ex. 300 nm. em. 378 nm and 750 nm (gel) |
| Hyperforin | 0.25 mg/mL in MeOH | 300 nm (gel and liquid) | ex. 300 nm, em. 378 nm and 750 nm (liquid); ex. 300 nm, em. 375 nm and 750 nm (gel) |
| Hypericin | 0.5 mg/mL in 1 M NaOH | 325 nm. 440 nm. 563 nm and 625 nm (gel): 345 nm. 455 nm and 620 nm (liquid) | ex. 460 nm, em, 700 nm (liquid); ex. 345 nm, em. 700 nm (liquid); ex. 620 nm, em. 700 nm (liquid); ex. 325 nm. em. 366 nm and 725 nm (gel) |
| Lycopene | 1 mg/mL in chloroform | 490 nm (gel): 460 nm, 485 nm and 520 nm (liquid) | ex. 300 nm, em. 386 nm and 750 nm; ex. 300 nm, em. 415 nm and 815 nm (gel) |
| Oleuropein | 10 mg/mL in DMSO | 325 nm (gel and liquid) | ex. 325 nm, em. 418 nm and 765nm (liquid); ex. 325 nm em. 380 nm and 750 nm (gel) |
| Piperine | Chloroform or EtOH | 344 nm | ex. 460 nm, em. 634 nm (in gel): ex. 340 nm, em. 443 nm |
| Resveratrol | 10 mg/mL in EtOH | 300 nm-325 nm | ex. 300 nm, em. 403 nm and 762 nm (liquid); ex. 300 nm. em. 403 nm and 773 nm (gel) |
| Sanguinarine chloride hydrate | 3.33 mg/mL in MeOH | 300 nm. 405 nm. and 470 nm (liquid): 330 nm. 405 nm and 477 nm (gel) | ex, 460 nm, em, 591 nm (in gel); ex. 460 nm, em. 600 nm (liquid); ex. 300 nm, em. 600 nm (liquid); ex. 405 nm, em. 600 nm (liquid); ex. 330 nm, em. 590 nm (gel); ex. 405 nm. em. 594 nm and 813 nm (gel) |
| Tannic acid | 10 mg/mL in $H_2O$ | -- | -- |

(continued)

| Compound | Solubilization | Absorbance peak (nm) | Emission peak (nm)** |
|---|---|---|---|
| Theobromine | Insoluble | -- | -- |
| Zeaxanthin | 2 mg/mL chloroform | 465 nm (gel and liquid) | ex. 300 nm, em. 405 nm and 740 nm Hiquid); ex. 300 nm, em. 415 nm. 432 nm, 740 nm and 817 nm (gel) |
| ** ex = excitation and em = emission | | | |

Example 2: Spectroscopy of Extracts of Curcuma

A series of various spectroscopy experiments were performed using an extract of Curcuma in order to evaluate the effect that this photosynthetic organism-derived extract comprising at least one chromophore has on the spectral properties of biophotonic compositions.

A) As a control, a carrier gel of approximately 2 mm thickness and containing urea peroxide but lacking any chromophore was exposed to an actinic light source (5 cm distance from the surface of the carrier gel), as detailed in the table below, and fluorescence readings recorded.

| Gel only | | mW/cm2 at 5cm | | | | | | | | | | | | |
|---|---|---|---|---|---|---|---|---|---|---|---|---|---|---|
| | | 0 | 0,5 min | 1 min | 1,5 min | 2 min | 2,5 min | 3 min | 3,5 min | 4 min | 4,5 min | 5 min | J/cm2 | |
| Lamp | 400-518 | 89.40 | 89.22 | 89.09 | 89.09 | 88.97 | 88.99 | 88.88 | 88.96 | 89.11 | 88.80 | 88.75 | 26.71 | 99.9% |
| Fluoresc. | 519-760 | 0.03 | 0.04 | 0.05 | 0.04 | 0.03 | 0.04 | 0.05 | 0.05 | 0.08 | 0.04 | 0.03 | 0.01 | 0.1% |
| total | 400-760 | 89.43233 | 89.25738 | 89.13575 | 89.13057 | 88.99553 | 89.03099 | 88.92131 | 89.00899 | 89.19097 | 88.84694 | 88.77427 | 26.73 | 100.0% |
| %fluorescence | | 0.0% | 0.0% | 0.1% | 0.0% | 0.0% | 0.0% | 0.1% | 0.1% | 0.1% | 0.0% | 0.0% | 0.00 | 0.1% |
| Purple | (400)-450 | 55.3360 | 54.8224 | 54.6446 | 54.5945 | 54.4153 | 54.3965 | 54.2227 | 54.3187 | 54.3972 | 54.0804 | 54.0140 | 16.36 | 61.2% |
| Blue | 450-500 | 33.9369 | 34.2466 | 34.2979 | 34.3507 | 34.4136 | 34.4454 | 34.4968 | 34.4887 | 34.5549 | 34.5856 | 34.5866 | 10.31 | 38.6% |
| Green | 500-570 | 0.1575 | 0.1881 | 0.1914 | 0.1845 | 0.1666 | 0.1871 | 0.2000 | 0.1945 | 0.2294 | 0.1809 | 0.1736 | 0.06 | 0.2% |
| Yellow | 570-591 | 0.0001 | 0.0003 | 0.0000 | 0.0008 | 0.0000 | 0.0000 | 0.0000 | 0.0000 | 0.0019 | 0.0000 | 0.0000 | 0.00 | 0.0% |
| Orange | 591-610 | 0.0000 | 0.0000 | 0.0000 | 0.0000 | 0.0000 | 0.0007 | 0.0000 | 0.0000 | 0.0000 | 0.0000 | 0.0000 | 0.00 | 0.0% |
| [illegible] | 610-760 | 0.0019 | 0.0000 | 0.0019 | 0.0000 | 0.0000 | 0.0014 | 0.0018 | 0.0070 | 0.0076 | 0.0000 | 0.0000 | [illegible] | 0.0% |
| total | (400-700) | 89.43 | 89.26 | 89.14 | 89.13 | 89.00 | 89.03 | 88.92 | 89.01 | 89.19 | 88.85 | 88.77 | 26.73 | 100.0% |

B) Into 25 g of carrier gel (containing urea peroxide, but lacking any chromophore) was mixed with one drop (0.05 mL) of Curcuma extract (St-Francis Herb Farm: curcuma extract in alcohol) and a 2 mm thickness of this gel was exposed to the actinic light source at a distance of 5 cm from the gel surface and the fluorescence readings were recorded.

| Curcuma tincture in UP Gel (one | | mW/cm2 at 5cm | | | | | | | | | | | | |
|---|---|---|---|---|---|---|---|---|---|---|---|---|---|---|
| | | 0 | 0,5 min | 1 min | 1,5 min | 2 min | 2,5 min | 3 min | 3,5 min | 4 min | 4,5 min | 5 min | J/cm2 | |
| Lamp | 400-518 | 11.75 | 16.16 | 20.18 | 25.05 | 29.46 | 34.06 | 37.70 | 41.14 | 43.44 | 46.22 | 48.91 | 9.16 | 99.2% |
| Fluoresc. | 519-760 | 0.67 | 0.29 | 0.42 | 0.18 | 0.27 | 0.24 | 0.14 | 0.14 | 0.11 | 0.10 | 0.09 | 0.08 | 0.8% |
| total | 400-760 | 12.42011 | 16.4542 | 20.5997 | 25.23098 | 29.73589 | 34.30102 | 37.84499 | 41.27898 | 43.54645 | 46.31677 | 48.99759 | 9.23 | 100.0% |
| %fluorescence | | 5.4% | 1.8% | 2.0% | 0.7% | 0.9% | 0.7% | 0.4% | 0.3% | 0.3% | 0.2% | 0.2% | 0.01 | 0.8% |
| Purple | (400)-450 | 5.5739 | 7.8491 | 10.0497 | 12.6834 | 15.1886 | 17.7324 | 19.7542 | 21.6676 | 22.9569 | 24.4900 | 25.9748 | 4.74 | 51.3% |
| Blue | 450-500 | 6.0093 | 8.1782 | 9.9691 | 12.2262 | 14.1141 | 16.1715 | 17.7964 | 19.3226 | 20.3267 | 21.5744 | 22.7780 | 4.37 | 47.3% |
| Green | 500-570 | 0.5190 | 0.3332 | 0.3911 | 0.2607 | 0.3177 | 0.2983 | 0.2490 | 0.2454 | 0.2338 | 0.2273 | 0.2201 | 0.09 | 1.0% |
| Yellow | 570-591 | 0.1169 | 0.0387 | 0.0702 | 0.0260 | 0.0461 | 0.0411 | 0.0233 | 0.0226 | 0.0178 | 0.0152 | 0.0159 | 0.01 | 0.1% |
| Orange | 591-610 | 0.0809 | 0.0254 | 0.0509 | 0.0116 | 0.0349 | 0.0284 | 0.0150 | 0.0153 | 0.0083 | 0.0085 | 0.0074 | 0.01 | 0.1% |
| [illegible] | 610-760 | 0.1237 | 0.0308 | 0.0709 | 0.0238 | 0.0360 | 0.0306 | 0.0076 | 0.0061 | 0.0031 | 0.0018 | 0.0016 | [illegible] | 0.1% |
| total | (400-700) | 12.42 | 16.46 | 20.60 | 25.23 | 29.74 | 34.30 | 37.85 | 41.28 | 43.55 | 46.32 | 49.00 | 9.23 | 100.0% |

C) Into 25 g of carrier gel (containing urea peroxide, but lacking any chromophore) was mixed with three drops (0.15 mL) of Curcuma extract (St-Francis Herb Farm:
curcuma extract in alcohol) and a 2 mm thickness of this gel was exposed to the actinic light source at a distance of 5 cm from the gel surface and the fluorescence readings were recorded.

| Curcuma tincture in UP Gel (3 d | | mW/cm2 at 5cm | | | | | | | | | | | | |
|---|---|---|---|---|---|---|---|---|---|---|---|---|---|---|
| | | 0 | 0,5 min | 1 min | 1,5 min | 2 min | 2,5 min | 3 min | 3,5 min | 4 min | 4,5 min | 5 min | J/cm2 | |
| Lamp | 400-518 | 3.55 | 5.44 | 7.17 | 9.57 | 12.09 | 14.59 | 17.12 | 19.66 | 22.19 | 24.20 | 26.59 | 4.07 | 97.4% |
| Fluoresc. | 519-760 | 0.63 | 0.50 | 0.48 | 0.38 | 0.37 | 0.33 | 0.30 | 0.11 | 0.23 | 0.21 | 0.20 | 0.11 | 2.5% |
| total | 400-760 | 4.178508 | 5.938783 | 7.649838 | 9.952764 | 12.46002 | 14.92856 | 17.42254 | 19.771 | 22.4242 | 24.40211 | 26.79235 | 4.17 | 100.0% |
| %fluorescence | | 15.0% | 8.5% | 6.3% | 3.8% | 2.9% | 2.2% | 1.7% | 0.6% | 1.0% | 0.8% | 0.8% | 0.03 | 2.5% |
| Purple | (400)-450 | 1.1642 | 1.9915 | 2.8060 | 3.9465 | 5.1983 | 6.4597 | 7.7567 | 9.0602 | 10.4011 | 11.4712 | 12.7589 | 1.83 | 43.3% |
| Blue | 450-500 | 2.2356 | 3.2931 | 4.2105 | 5.4723 | 6.7415 | 7.9792 | 9.2083 | 10.4600 | 11.6389 | 12.5698 | 13.6731 | 2.21 | 53.0% |
| Green | 500-570 | 0.4727 | 0.4192 | 0.4009 | 0.3634 | 0.3522 | 0.3346 | 0.3228 | 0.2252 | 0.2911 | 0.2775 | 0.2787 | 0.10 | 2.5% |
| Yellow | 570-591 | 0.1059 | 0.0894 | 0.0801 | 0.0647 | 0.0603 | 0.0550 | 0.0501 | 0.0138 | 0.0371 | 0.0340 | 0.0322 | 0.02 | 0.4% |
| Orange | 591-610 | 0.0732 | 0.0590 | 0.0564 | 0.0443 | 0.0427 | 0.0389 | 0.0350 | 0.0090 | 0.0284 | 0.0245 | 0.0231 | 0.01 | 0.3% |
| Red | 610-760 | 0.1301 | 0.0889 | 0.0983 | 0.0634 | 0.0667 | 0.0628 | 0.0512 | 0.0031 | 0.0288 | 0.0261 | 0.0273 | [illegible] | 0.4% |
| total | (400-700) | 4.18 | 5.94 | 7.65 | 9.95 | 12.46 | 14.93 | 17.42 | 19.77 | 22.43 | 24.40 | 26.79 | 4.17 | 100.0% |

As can be observed from these data, adding Curcuma to the gel increased the amount of red, orange and yellow light emitted from the gel following exposure to actinic light.

D) 25 g of carrier gel (as per Example A, above) was mixed with 10% (w/w) of pHEMA powder containing contains 1:1 ratio of Eosin Y/Fluorescein (the pHEMA powder containing the chromophores was prepared from a 100 g block of pHEMA containing 100 g of Eosin Y plus 100 g of Fluoroscein). A 2 mm thickness of this gel was exposed to the actinic light source at a distance of 5 cm from the gel surface and the fluorescence readings were recorded.

| UP Gel with 10% PHEMA | | mW/cm2 at 5cm | | | | | | | | | | | | |
|---|---|---|---|---|---|---|---|---|---|---|---|---|---|---|
| | | 0 | 0,5 min | 1 min | 1,5 min | 2 min | 2,5 min | 3 min | 3,5 min | 4 min | 4,5 min | 5 min | J/cm2 | |
| Lamp | 400-518 | 59.79 | 59.72 | 61.94 | 64.04 | 65.01 | 65.30 | 65.38 | 65.46 | 65.47 | 65.31 | 65.50 | 19.12 | 99.4% |
| Fluoresc. | 519-760 | 1.32 | 0.64 | 0.38 | 0.21 | 0.18 | 0.17 | 0.17 | 0.17 | 0.19 | 0.15 | 0.17 | 0.11 | 0.6% |
| total | 400-760 | 61.11501 | 60.3627 | 62.32099 | 64.25519 | 65.191 | 65.47371 | 65.55132 | 65.62872 | 65.65456 | 65.45787 | 65.6653 | 19.23 | 100.0% |
| %fluorescence | | 2.2% | 1.1% | 0.6% | 0.3% | 0.3% | 0.3% | 0.3% | 0.3% | 0.3% | 0.2% | 0.3% | 0.01 | 0.6% |
| Purple | (400)-450 | 39.4744 | 37.3823 | 38.0532 | 38.7097 | 38.9826 | 39.1127 | 39.1663 | 39.1579 | 39.1872 | 39.0122 | 39.1551 | [illegible] | 60.6% |
| Blue | 450-500 | 20.3185 | 22.3303 | 23.8432 | 25.2601 | 25.9398 | 26.1062 | 26.1278 | 26.2184 | 26.2052 | 26.2157 | 26.2618 | 7.46 | 38.8% |
| Green | 500-570 | 0.7771 | 0.4243 | 0.2997 | 0.2164 | 0.2070 | 0.1936 | 0.2043 | 0.1963 | 0.2058 | 0.1846 | 0.1995 | 0.09 | 0.5% |
| Yellow | 570-591 | 0.3851 | 0.1533 | 0.0860 | 0.0444 | 0.0415 | 0.0387 | 0.0364 | 0.0374 | 0.0375 | 0.0308 | 0.0350 | 0.03 | 0.1% |
| Orange | 591-610 | 0.1476 | 0.0643 | 0.0352 | 0.0199 | 0.0184 | 0.0186 | 0.0148 | 0.0165 | 0.0171 | 0.0137 | 0.0136 | 0.01 | 0.1% |
| Red | 610-760 | 0.0157 | 0.0100 | 0.0048 | 0.0053 | 0.0021 | 0.0044 | 0.0021 | 0.0026 | 0.0022 | 0.0012 | 0.0005 | [illegible] | 0.0% |
| total | (400-700) | 61.12 | 60.36 | 62.32 | 64.26 | 65.19 | 65.47 | 65.55 | 65.63 | 65.65 | 65.46 | 65.67 | 19.23 | 100.0% |

E) 25 g of carrier gel (as per Example A, above) was mixed with 10% (w/w) of pHEMA powder containing 1:1 ratio of Eosin Y/Fluorescein (the pHEMA powder containing the chromophores was prepared from a 100 g block of pHEMA containing 100 g of Eosin Y plus 100 g of Fluorescein) and three drops (0.15 mL) of Curcuma extract (St-Francis Herb Farm: curcuma extract in alcohol). A 2 mm thickness of this gel was exposed to the actinic light source at a distance of 5 cm from the gel surface and the fluorescence readings were recorded.

| Gel + PHEMA (powder) +3 drops | | mW/cm2 at 5cm | | | | | | | | | | | | |
|---|---|---|---|---|---|---|---|---|---|---|---|---|---|---|
| | | 0 | 0,5 min | 1 min | 1,5 min | 2 min | 2,5 min | 3 min | 3,5 min | 4 min | 4,5 min | 5 min | J/cm2 | |
| Lamp | 400-518 | 0.36 | 0.50 | 0.70 | 1.21 | 1.81 | 2.52 | 3.37 | 4.13 | 4.64 | 5.92 | 6.86 | 0.75 | 65.0% |
| Fluoresc. | 519-760 | 1.90 | 1.76 | 1.63 | 1.45 | 1.28 | 1.23 | 1.10 | 1.09 | 1.06 | 0.96 | 0.94 | 0.40 | 34.8% |
| total | 400-760 | 2.263431 | 2.258443 | 2.329047 | 2.659867 | 3.087271 | 3.749139 | 4.467285 | 5.221597 | 5.696651 | 6.880531 | 7.792873 | 1.16 | 99.8% |
| %fluorescence | | 84.1% | 77.9% | 70.1% | 54.7% | 41.5% | 32.7% | 24.6% | 20.9% | 18.6% | 13.9% | 12.0% | 0.35 | 34.9% |
| Purple | (400)-450 | 0.1061 | 0.1536 | 0.2247 | 0.4188 | 0.6589 | 0.9669 | 1.3352 | 1.6862 | 1.9210 | 2.5238 | 2.9831 | 0.30 | 25.8% |
| Blue | 450-500 | 0.2259 | 0.3145 | 0.4381 | 0.7461 | 1.1006 | 1.5055 | 1.9774 | 2.3856 | 2.6530 | 3.3342 | 3.8052 | 0.44 | 37.9% |
| Green | 500-570 | 0.6845 | 0.6427 | 0.6094 | 0.5688 | 0.5360 | 0.5247 | 0.4966 | 0.4995 | 0.4952 | 0.4610 | 0.4680 | 0.17 | 14.3% |
| Yellow | 570-591 | 0.4681 | 0.4204 | 0.3798 | 0.3262 | 0.2874 | 0.2650 | 0.2408 | 0.2295 | 0.2213 | 0.1980 | 0.1915 | 0.09 | 7.8% |
| Orange | 591-610 | 0.3040 | 0.2771 | 0.2533 | 0.2204 | 0.1934 | 0.1806 | 0.1619 | 0.1567 | 0.1517 | 0.1382 | 0.1309 | 0.06 | 5.3% |
| Red | 610-760 | 0.4868 | 0.4612 | 0.4339 | 0.3885 | 0.3187 | 0.3138 | 0.2620 | 0.2704 | 0.2606 | 0.2311 | 0.2195 | [illegible] | 8.9% |
| total | (400-700) | 2.28 | 2.27 | 2.34 | 2.67 | 3.10 | 3.76 | 4.47 | 5.23 | 5.70 | 6.89 | 7.80 | 1.16 | 100.0% |

As can be observed from these data, adding Curcuma to the gel increased the amount of red, yellow and orange light emitted from the gel following exposure to actinic light.

Example 3: Treatment of a Patient Afflicted with Post-Surgical Scarring with a Biophotonic Composition of the Disclosure

A biophotonic composition disclosed herein can be prepared for treating a patient with post-surgical scarring. In an exemplary embodiment, the biophotonic composition can be prepared by grinding a photosynthetic organism into a powder. The powder is mixed with a suitable organic solvent, for a minimum of, e.g., an hour, up to about 3 days. Suitable organic solvents include, e.g., ethanol, ethylene glycol, or propylene glycol.

The biophotonic composition of the disclosure is prepared by mixing a photosynthetic organism-derived chromophore (e.g., the extract prepared above) and a carbopol carrier gel comprising urea peroxide. The resulting composition is applied to the tissue of a patient with post-surgical scarring and activated with actinic light provided by a LED photocuring device. The composition is removed following treatment.

## Claims

1. A biophotonic composition comprising at least one photosynthetic organism-derived chromophore and at least one at least one xanthene dye and a carrier medium, wherein the at least one photosynthetic organism-derived chromophore is selected from: aloe-emodin, apigenin, berberine, caffeic acid, caffeine, curcumin, garcinia acid, gingerol, hyperforin, hypericin, ellagic acid, lycopene, oleuropein, piperine, resveratrol, sanguinarine, tannic acid, theobromine, zeaxanthin and combinations thereof

    wherein the at least one photosynthetic organism-derived chromophore or the at least one xanthene dye is a fluorescent chromophore; and
    wherein the at least one photosynthetic organism-derived chromophore or the at least one xanthene dye has an emission spectrum that overlaps at least 20% with an absorption spectrum of the at least second chromophore.

2. The biophotonic composition of claim 1, further comprising an oxidant.

3. The biophotonic composition of claim 2, wherein the oxidant is carbamide peroxide.

4. The biophotonic composition of any one of claims 1 to 3, wherein the carrier medium comprises one or more of a hydrophilic polymer, a hygroscopic polymer, or a hydrated polymer.

5. The biophotonic composition of one of claims 1 to 4, wherein the at least one photosynthetic organism-derived chromophore or the at least one xanthene dye absorbs and/or emits light within the visible range.

6. The biophotonic composition of claim 1, wherein the at least one photosynthetic organism-derived chromophore or the at least one xanthene dye absorbs and/or emits light within the green, orange and yellow portions of the electromagnetic spectrum.

7. The biophotonic composition of one of claims 1 to 6, wherein the xanthene dye is Eosin Y, Eosin B, Erythrosin B, Fluorescein, Rose Bengal, Phloxin B, or combinations thereof.

8. The biophotonic composition of any one of claims 1 to 7, wherein the at least one photosynthetic organism-derived chromophore or the at least one xanthene dye is present in an amount of between about 0.0001% to about 40% by weight of the total composition, or between about 0.0001% to about 2% by weight of the total composition.

9. The biophotonic composition of any one of claims 1 to 8, further comprising a chromophore-protecting agent.

10. Cosmetic, non-therapeutic use of the biophotonic composition of any of claims 1 to 9, whereby the treatment is optionally selected from skin rejuvenation and conditioning.

11. The biophotonic composition of any of claims 1 to 9 for use in a medical treatment of tissue or bone, whereby the treatment is optionally selected from wound healing, oral disease treatment, periodontitis treatment, treatment of bacterial, viral or fungal infections, treatment of a fistula, treatment of a skin condition, and bone regeneration.

## Patentansprüche

1. Biophotonische Zusammensetzung, umfassend mindestens einen von einem photosynthetischen Organismus abgeleitete Chromophor und mindestens einen Xanthenfarbstoff und ein Trägermedium, wobei der mindestens eine von einem photosynthetischen Organismus abgeleitete Chromophor ausgewählt ist aus: Aloe-Emodin, Apigenin, Berberin, Kaffeesäure, Koffein, Curcumin, Garciniasäure, Gingerol, Hyperforin, Hypericin, Ellagsäure, Lycopin, Oleuropein, Piperin, Resveratrol, Sanguinarin, Gerbsäure, Theobromin, Zeaxanthin und Kombinationen davon,

    wobei der mindestens eine aus einem photosynthetischen Organismus abgeleitete Chromophor oder der mindestens eine Xanthenfarbstoff ein fluoreszierender Chromophor ist; und
wobei der mindestens eine aus einem photosynthetischen Organismus abgeleitete Chromophor oder der mindestens eine Xanthenfarbstoff ein Emissionsspektrum aufweist, das sich zu mindestens 20% mit einem Absorptionsspektrum des mindestens zweiten Chromophors überlappt.

2. Biophotonische Zusammensetzung nach Anspruch 1, die weiter ein Oxidationsmittel umfasst.

3. Biophotonische Zusammensetzung nach Anspruch 2, wobei das Oxidationsmittel Carbamidperoxid ist.

4. Biophotonische Zusammensetzung nach einem der Ansprüche 1 bis 3, wobei das Trägermedium ein oder mehrere von einem hydrophilen Polymer, hygroskopischen Polymer oder hydratisierten Polymer umfasst.

5. Biophotonische Zusammensetzung nach einem der Ansprüche 1 bis 4, wobei der mindestens eine von einem photosynthetischen Organismus abgeleitete Chromophor oder der mindestens eine Xanthenfarbstoff Licht innerhalb des sichtbaren Bereichs absorbiert und/oder emittiert.

6. Biophotonische Zusammensetzung nach Anspruch 1, wobei der mindestens eine von einem photosynthetischen Organismus abgeleitete Chromophor oder der mindestens eine Xanthenfarbstoff Licht innerhalb des grünen, orangen und gelben Abschnitts des elektromagnetischen Spektrums absorbiert und/oder emittiert.

7. Biophotonische Zusammensetzung nach einem der Ansprüche 1 bis 6, wobei der Xanthenfarbstoff Eosin Y, Eosin B, Erythrosin B, Fluorescein, Rose Bengal, Phloxin B oder Kombinationen davon ist.

8. Biophotonische Zusammensetzung nach einem der Ansprüche 1 bis 7, wobei der mindestens eine von einem photosynthetischen Organismus abgeleitete Chromophor oder der mindestens eine Xanthenfarbstoff in einer Menge von zwischen etwa 0,0001 % bis etwa 40 Gew.-% der Gesamtzusammensetzung oder von zwischen etwa 0,0001 % bis etwa 2 Gew.-% der Gesamtzusammensetzung vorliegt.

9. Biophotonische Zusammensetzung nach einem der Ansprüche 1 bis 8, die weiter ein Chromophor-Schutzmittel umfasst.

10. Kosmetische, nicht-therapeutische Verwendung der biophotonischen Zusammensetzung nach einem der Ansprüche 1 bis 9, wobei die Behandlung wahlweise aus Hautverjüngung und Hauptpflege ausgewählt ist.

11. Biophotonische Zusammensetzung nach einem der Ansprüche 1 bis 9 zur Verwendung bei einer medizinischen Behandlung von Gewebe oder Knochen, wobei die Behandlung wahlweise aus der Wundheilung, der Behandlung von Mundkrankheiten, der Behandlung von Parodontitis, der Behandlung von bakteriellen, viralen oder Pilzinfektionen, der Behandlung einer Fistel, der Behandlung einer Hautkrankheit und der Knochenregeneration ausgewählt ist.

## Revendications

1. Composition biophotonique comprenant au moins un chromophore dérivé d'un organisme photosynthétique et au moins un colorant au xanthène et un milieu porteur, dans lequel l'au moins un chromophore dérivé d'un organisme photosynthétique est sélectionné parmi : l'aloe-émodine, l'apigénine, la berbérine, l'acide caféique, la caféine, la curcumine, l'acide garcinique, le gingérol, l'hyperforine, l'hypéricine, l'acide ellagique, le lycopène, l'oleuropéine, la pipérine, le resvératrol, la sanguinarine, l'acide tannique, la théobromine, la zéaxanthine et des combinaisons de ceux-ci

dans laquelle l'au moins un chromophore dérivé d'un organisme photosynthétique ou l'au moins un colorant au xanthène est un chromophore fluorescent ; et
dans laquelle l'au moins un chromophore dérivé d'un organisme photosynthétique ou l'au moins un colorant au xanthène a un spectre d'émission qui chevauche au moins 20 % d'un spectre d'absorption de l'au moins un second chromophore.

2. Composition biophotonique selon la revendication 1, comprenant en outre un oxydant.

3. Composition biophotonique selon la revendication 2, dans laquelle l'oxydant est du peroxyde d'urée.

4. Composition biophotonique selon l'une quelconque des revendications 1 à 3, dans laquelle le milieu porteur comprend un ou plusieurs parmi un polymère hydrophile, un polymère hygroscopique ou un polymère hydraté.

5. Composition biophotonique selon l'une des revendications 1 à 4, dans laquelle l'au moins un chromophore dérivé d'un organisme photosynthétique ou l'au moins un colorant au xanthène absorbe et/ou émet une lumière au sein de la plage visible.

6. Composition biophotonique selon la revendication 1, dans laquelle l'au moins un chromophore dérivé d'un organisme photosynthétique ou l'au moins un colorant au xanthène absorbe et/ou émet une lumière au sein des portions verte, orange et jaune du spectre électromagnétique.

7. Composition biophotonique selon l'une des revendications 1 à 6, dans laquelle le colorant au xanthène est l'éosine Y, l'éosine B, l'érythrosine B, la fluorescéine, le rose bengale, la phloxine B ou des combinaisons de ceux-ci.

8. Composition biophotonique selon l'une quelconque des revendications 1 à 7, dans laquelle l'au moins un chromophore dérivé d'un organisme photosynthétique ou l'au moins un colorant au xanthène est présent en une quantité entre environ 0,0001 % et environ 40 % en poids de la composition totale, ou entre environ 0,0001 % et environ 2 % en poids de la composition totale.

9. Composition biophotonique selon l'une quelconque des revendications 1 à 8, comprenant en outre un agent protecteur de chromophore.

10. Utilisation cosmétique non-thérapeutique de la composition biophotonique selon l'une quelconque des revendications 1 à 9, moyennant quoi le traitement est facultativement sélectionné parmi le rajeunissement et le conditionnement cutanés.

11. Composition biophotonique selon l'une quelconque des revendications 1 à 9 pour une utilisation dans un traitement médical de tissus ou d'os, dans lequel le traitement est facultativement sélectionné parmi une cicatrisation de plaies, un traitement oral de maladie, un traitement de périodontite, un traitement d'infections bactériennes, virales ou fongiques, un traitement d'une fistule, un traitement d'une affection cutanée et une régénération osseuse.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 20170410177 A
- AU 2013200644
- US 2015119788 A
- US 20040009227
- US 20110081530
- US 3141321 A
- US 4402959 A
- US 4430381 A
- US 4533435 A
- US 4625026 A
- US 4736467 A
- US 4855139 A
- US 5069907 A
- US 5091102 A
- US 5639464 A
- US 5853883 A
- US 5854147 A
- US 5894042 A
- US 5919554 A
- US 20040009227 A
- US 20110081530 A
- US 20090069217
- US 7598291 B
- US 7722904 B
- US 6203805 B
- US 5529769 A
- US 20060247313
- US 20080108681
- US 20110130459
- US 20090325885
- US 20110086060
- US 2011008600
- US 20090220450 A